# EUROPEAN PATENT APPLICATION

(11) **EP 4 393 451 A2**
(43) Date of publication of application: **03.07.2024**
(21) Application number: 24164199.2
(22) Date of filing: 18.08.2021
(51) Int. Cl.: A61F 2/07

(54) **STENT WITH ENHANCED LOW CRIMPING PROFILE**

(30) Priority: 17.08.2021 US 202117404879
(62) Divisional of application: 21790980.3
(71) Applicant: Medinol Ltd., Tel Aviv 6158101 (IL)
(72) Inventor: RICHTER, Yoram, 4704247 Ramat Hasharon (IL); BELOBROVY, Igor, 4922351 Petah-Tiqwa (IL); DAVID, Yaron, New York, 10021 (US); WEIZMANN, Oleg, 4636431 Herzliya (IL)
(74) Representative: Kuhnen & Wacker Patent- und Rechtsanwaltsbüro PartG mbB

(57) **Abstract**

The invention relates to an endovascular device (100), comprising: a main stent component (105) having a tubular shape, a first end (105a) and a second end (105b), wherein the main stent component (105) comprises: a helical stent pattern having a plurality of continuous windings such that the plurality of windings (115) are oriented in a helical direction (H) of the endovascular device (100), wherein each winding of the plurality of windings (115) comprises two interconnected bands (115A-B), and wherein at least two adjacent windings (115) are interconnected in the longitudinal direction of the stent by a flexible connection (2702, 2802).

## Description

### FIELD OF THE INVENTION

The invention relates generally to intraluminal endovascular devices, such as stents which are implanted into vessels within the body, for example blood vessels, in order to open vessels that were narrowed or blocked as a result of, for example, coronary artery disease (CAD), restore blood flow, and/or maintain the vessels' patency. More particularly, the invention relates to endovascular devices, including stents, having a reduced compressed or crimped profile and/or an enlarged expanded profile.

### BACKGROUND OF THE INVENTION

Various stents are known in the art. Typically, stents are mesh-like structures, tubular in shape, and are expandable from a smaller, unexpanded, diameter to a larger, expanded, diameter. For implantation, the stent is typically mounted on the distal part of a catheter with the stent being held on the catheter in a crimped, unexpanded diameter. Using a catheter guided by a guide-wire slidably extending therethrough, the unexpanded stent is delivered through the vascular or gastro-intestinal system to the intended implantation site, for example a blood vessel or a coronary artery. Once the stent is at the intended implantation site, it is expanded radially, typically either by a force, for example by inflating a balloon on the inside of the stent, or by allowing the stent to self-expand, for example by removing a sleeve from around a self-expanding stent thus allowing the stent to expand radially. In either case, the expanded stent resists the tendency of the vessel to re-narrow, thereby maintaining the vessel's patency.

Stents may be manufactured by laser cutting the stent pattern into a tube or a flat sheet of metal. In the latter case, the sheet is later rolled and fixed such as by welding, mechanical lock or otherwise, to form the tubular structure of the stent.

One type of stent is known as the helical or coiled stent. Such a stent design is described in, for example, U.S. Pat. Nos. 6,503,270 and 6,355,059, which are incorporated herein, *in toto,* by reference. This stent design is configured as a helical stent in which the coil is formed from a wound strip of cells, where the cells form a serpentine pattern comprising a series of bends formed from an alternating arrangement of struts connected to loops. Other similar helically coiled stent structures are known in the art, such as the stent design described in, for example, U.S. Pat. Nos. 8,382,821; 9,456,910; 9,155,639; 9,039,755 and 9,603,731 which are incorporated herein, *in toto,* by reference. These stent designs are configured as helical stents in which the coil is formed from a flat or tubular metal where the cells are formed from an undulating pattern of the helically wound coil.

In prior art stents, there typically is a tradeoff between longitudinal (or axial) flexibility and radial strength, as well as the ability to tightly compress or crimp the stent onto a catheter so that the stent can be more easily delivered through narrow tortuous vasculature (*e.g*., small side branches) and so that it does not move relative to the catheter or dislodge prematurely prior to controlled implantation in a vessel. Prior art stent designs also typically have a tradeoff between providing sufficient radial strength when the stent is expanded so that it can sufficiently support the vessel's lumen, and providing sufficient longitudinal flexibility so it can easily conform to the natural curvature of the vessel.

The crimped or compressed diameter of prior art stents is limited due to interference between adjacent struts, adjacent loops and/or a combination thereof. In addition, in self-expandable stents, interference between adjacent struts and/or loops may subject a portion of a strut to high stress/strain concentrations which may prevent the stent from fully expanding when deployed. For example, if a self-expanding stent is compressed beyond its elastic limit in an attempt to provide a smaller outside diameter, the stent will not return to its desired deployed expanded diameter due to permanent deformation.

Therefore, a continued need exists in the art for a stent having simultaneously sufficient radial strength, high degree of longitudinal flexibility and conformability to the vessel's natural curvature and movements, as well as a stent having a reduced compressed profile for enhanced delivery through small diameter or tortuous vessels (such as in side branches of the coronary vessel anatomy) and an enlarged expanded profile for deployment in large diameter vessels (such as in main branches of the coronary vessel anatomy), and while maintaining low stress/strain concentrations on portions of the stent. Thus, a need exists for a stent having an enlarged expanded diameter and a reduced compressed diameter in order to allow the stent to be used in any clinical situation compared to a conventional stent, while achieving optimal stress/strain distribution along the stent. Further, a need exists to limit the interference between adjacent struts and/or loops in the compressed profile in order to achieve a reduced compressed profile and a reduced stress/strain concentrations, as well as to minimize harmful interaction between adjacent struts. Minimizing harmful interactions between adjacent struts includes, for example, (a) reducing damage to stent coatings caused by contact between adjacent struts, (b) reducing the likelihood of damage to a balloon of a balloon catheter due to a material of the balloon being pinched between adjacent struts, (c) reducing stress imparted on the struts caused by the interaction between the adjacent struts, (d) reducing the force required for crimping due to fewer strut-to-strut interactions, and/or (e) reducing the physical limits on the stent that limit the stent from being compressed (crimped) any further.

### SUMMARY OF THE INVENTION

The invention relates to a stent having an enlarged expanded diameter and/or a reduced compressed diameter, such that the stent has reduced outside diameter in its compressed state compared to conventional stents. The stent of the invention comprises a bent strut design in the crimped profile of the stent which reduces the compressed outside diameter compared to a compressed outside diameter of any given conventional stent. Any reduction of the compressed outside diameter, *i.e*., the crimping profile, is clinically significant and allows for enhanced crimping.

In one aspect, the invention relates to an endovascular device comprising a main stent component having a tubular shape and a first end and a second end, said device having a first radiopaque marker having a shape with at least two distinct profiles when viewed from different angles said marker attached to the main stent component, and a second radiopaque marker having a shape with at least two distinct profiles when viewed from different angles said marker attached to the main stent component wherein the first and second radiopaque markers are positioned on the main stent component to be offset by less than 180 degrees relative to the other. This aspect of the invention is independent of stent design.

To enhance X-ray visibility of the stent, the stent of the invention may comprise a plurality of radiopaque markers mounted at each end of the stent. The plurality of radiopaque markers at each end are offset relative to one another such that the plurality of radiopaque markers have a shape with at least two different profiles. These features make the stent advantageously observed during angiographic and/or radiographic imaging to enable improved stent navigation and placement within a vessel. The plurality of radiopaque markers at each end of the stent are offset by less than 180 degrees relative to another marker on the same end of the stent.

In a second aspect of the invention, the stent according to the invention comprises an inventive structure having at least a portion which is spiral in structure in a main stent component having a first end portion and a second end portion. The main stent component comprises an undulating pattern of struts connected by loops. In one embodiment, at least one strut of the stent comprises one or more bends, curves or undulations in the strut design in at least the compressed configuration of the stent. For example, the bent strut design may include first and second bent, curved or angled sections facing in opposite convex and concave orientations. These opposing bends or angles join together at one or more locations along the strut. As the stent is compressed, a loop-oppositely aligned with a bent section (*e.g*., the first or second bent sections)-is moved a desired distance closer to the opposing bent section to form a nestled arrangement and achieve a desired smaller compressed diameter than in conventional stents. For example, the loop and the opposing bent section may be moved or compressed to substantially contact each other, where substantially contact is defined as a loop contacting or being in near contact with a bent section of the opposing strut. The nestled arrangement in the compressed configuration of the stent advantageously achieves a lower crimped profile than in conventional stents because the bend in the strut creates a space into which an opposing loop may nestle in the crimped orientation.

Adjacent loops in the helical direction may be axially offset with respect to an axis perpendicular to the lengthwise direction to form a staggered pattern of alignment of adjacent loops such that a loop is positioned to align with an end of an adjacent strut in the helical direction. In one embodiment, the staggered pattern of alignment of adjacent loops is positioned such that, as the stent is compressed to the crimped delivery diameter, the loops adjacent to the first and second bent sections in the helical direction are positioned to align with and nestle in the first and second bent sections, respectively, to form a nestled arrangement. The nestled arrangement may be such that the loops adjacent to the first and second bent sections in the helical direction contact or are in near-contact with the first and second bent sections, respectively, when the stent is compressed to the crimped delivery diameter.

A strut may have a varying width, *e.g.*, a width near a mid-section of the strut is smaller than a width near ends of the strut, or *vice versa.* In this embodiment, a width of the loop may be greater than a width of any portion of the strut.

The stent comprises the main stent component having a tubular shape and extending from a first end to a second end along a lengthwise direction of the stent. The main stent component may comprise a plurality of windings having a crimped delivery diameter and an expanded implanted diameter. A winding may comprise any number of bands as desired for a particular application. For example, a winding may comprise a single band, or may comprise two, three, or four or more bands which may be interconnected as desired to form cells within the winding. Any number of interconnections (e.g., an indirect connection such as a longitudinal connection, or a direct connection) may be provided as desired for a particular application, where the number of interconnections is indirectly proportional to the overall flexibility of the stent. In the embodiment in which the winding comprises a single band, no interconnection may exist between adjacent windings such that cells formed between adjacent windings are open cells that do not enclose a space therein. Alternatively, in the single band embodiment, interconnections may exist between some or all of the adjacent windings. In the embodiment in which the individual winding comprises a plurality of bands, an interconnection exists between the bands such that cells formed between adjacent interconnected bands within an individual winding are enclosed cells enclosing a space therein. In this embodiment in which an individual winding comprises a plurality of interconnected bands, adjacent windings may or may not include interconnections there-between. This aspect of the invention advantageously achieves a low crimping profile, which is advantageous lower as compared to the crimping profile of conventional stents.

The stent may further comprise a link interconnecting the two interconnected bands of each winding in the lengthwise direction. The two interconnected bands of each winding may be interconnected by a direct connection. In one embodiment, the link may be a straight connector and may extend in a gap between the two interconnected bands. The link and/or direct connection may connect first and second bands of the winding at loops on the first and second bands at a position where the gap between the interconnected bands is the shortest distance. The loops at which the first and second bands of a winding are connected are also referred to as attachment loops.

In another embodiment, the stent may further comprise a first end ring positioned at the first end and a second end ring positioned at the second end of the main stent component, where the first and second end rings may extend from the winding adjacent thereto. The first and second end rings may form approximately a right-angled cylinder at lengthwise ends of the stent. Each end ring may comprise one or more circumferential end bands interconnected in the lengthwise direction and may comprise the undulating pattern of loops coupled to pairs of struts. Similar to the windings of the main stent component, the circumferential end bands may be interconnected by a link and/or direct connection. In one embodiment, the transition between the windings of the main stent component and the first and second end rings may include at least one transition cell formed by the main stent component and one of the first and second end rings. The backbone of stent including the first and second end rings may collectively be referred to as the main stent component.

Further, similar to the main stent component, in one embodiment, the struts of the first and second end rings may have variable lengths to produce axially offset loops in the circumferential direction. Alternatively or in addition, the struts of the first and second end rings may have a variable width along the strut length, similar to that described with respect to the struts of the main stent component. Alternatively or in addition, the loops of the first and second end rings may have a width different from (*e.g.*, larger or smaller) a width of any portion of the strut.

The first and second end rings may also similarly comprise at least one bent strut, where, as the stent is compressed to the crimped delivery diameter, at least one loop is positioned to align with and nestle in one of the first and second bent sections of the bent strut adjacent to the loop in the circumferential direction. In one embodiment, all of the struts of the first and second end rings may have bent struts. In another embodiment, the first and second end rings may have a mixed strut design, such that some of the struts are bent struts and some of the struts are linear struts. In yet another embodiment, the first and second end rings do not comprise a bent strut, but rather have linear struts.

Further, in one embodiment, adjacent windings of the main stent component and/or the end rings may be unconnected by a link or direct connection such that no enclosed cells are formed between adjacent windings along the coiled pattern of the stent. In one exemplary embodiment, the main stent component comprises two interconnected bands within an individual winding thereby forming enclosed cells within each winding, and further comprises adjacent windings that are unconnected, in the longitudinal direction of the stent, by a link or direct connection such that no enclosed cells are formed between adjacent windings along the coiled pattern of the stent. This structure of a plurality of interconnected bands within an individual winding while adjacent windings are unconnected (i.e., no metallic indirect links or metallic direct connections in the longitudinal direction) allows for exceptional improved flexibility, preventing vessel straightening and allowing vessel flexion within, for example, the cardiac cycle.

In another embodiment, one or more connections (e.g., direct or indirect connections) may exist between the adjacent windings of the main stent component and/or the end rings. In this embodiment, some or all of the adjacent windings may be connected by an indirect connection, a direct connection, or a combination thereof. An indirect connection may be flexible metallic connector such as a link or cross-strut extending between adjacent bands of adjacent windings. For example, an indirect connection may extend between a first band of a first winding and a second band of a second winding, where the second winding is adjacent the first winding and the first band is adjacent the second band. In one embodiment, the indirect connection connects adjacent struts of adjacent windings, and may be referred to as an S-shaped connection. The struts (of the undulating pattern) at which the adjacent bands of adjacent windings are connected by the S-shaped connection may be referred to as attachment struts. A direct connection may directly connect adjacent bands of adjacent windings at loops of the undulating pattern, which may be referred to as attachment loops of adjacent windings. Such a direct connection between loops of adjacent windings may be referred to as an H-shaped connection. The direct connection may be achieved by any type of direct connection means, such as, fusing, welding, adhesive bonding, soldering, laser welding, mechanical joining, among others. The flexible connectors between adjacent windings are preferably sparse so as not to have a substantial effect on the longitudinal (i.e., axial) flexibility of the stent. The purpose of the flexible connectors (indirect or direction connectors) between adjacent windings is to enhance the axial stability of the stent.

### BRIEF DESCRIPTION OF THE DRAWINGS

FIG. 1 illustrates a flat view of a stent in the as-cut configuration according to one embodiment of the invention.
FIG. 2 is an enlarged view of an enclosed cell of the main stent structure of the stent of FIG. 1.
FIG. 3 is an enlarged view of a pair of struts connected to a loop of a stent in the as-cut configuration according to another embodiment of the invention.
FIG. 4 illustrates a 3-dimensional model of the stent, according to the embodiment of FIG. 3, from a first perspective and in the as-cut configuration.
FIG. 5 illustrates a 3-dimensional model of the stent according to FIG. 4 from a second perspective and in the as-cut configuration.
FIG. 6 illustrates a 3-dimensional model of the stent according to FIG. 4 from the first perspective and in the as-cut configuration.
FIG. 7 illustrates a 3-dimensional model of the stent according to FIG. 5 from the second perspective and in the as-cut configuration.
FIG. 8 illustrates a 3-dimensional model of the stent according to FIG. 4 from the third perspective and in the as-cut configuration.
FIG. 9 illustrates a 3-dimensional model of the stent according to FIG. 8 from the third perspective and in the as-cut configuration.
FIG. 10 illustrates the stent of FIG. 4 in a crimped, delivery configuration.
FIG. 11 illustrates the stent of FIG. 5 in the crimped, delivery configuration.
FIG. 12 illustrates the stent of FIG. 6 in the crimped, delivery configuration.
FIG. 13 illustrates the stent of FIG. 7 in the crimped, delivery configuration.
FIG. 14 illustrates the stent of FIG. 8 in the crimped, delivery configuration.
FIG. 15 illustrates the stent of FIG. 9 in the crimped, delivery configuration.
FIG. 16 illustrates the stent of FIG. 4 in an expanded, deployed configuration.
FIG. 17 illustrates the stent of FIG. 5 in the expanded, deployed configuration.
FIG. 18 illustrates the stent of FIG. 6 in the expanded, deployed configuration.
FIG. 19 illustrates the stent of FIG. 7 in the expanded, deployed configuration.
FIG. 20 illustrates the stent of FIG. 8 in the expanded, deployed configuration.
FIG. 21 illustrates the stent of FIG. 9 in the expanded, deployed configuration.
FIG. 22 illustrates a stent, in a tubular view, according to any of the embodiments of the present invention in a crimped configuration and having a polymer coating.
FIG. 23 illustrates a stent, in a tubular view, according to the embodiment of FIG. 22 in a radially expanded configuration and having a polymer coating.
FIG. 24 illustrates partial perspective view of a stent having two radiopaque markers offset relative to each other, according to the another embodiment of present invention.
FIG. 25A illustrates a planar view of the stent according to FIG. 24 along the length of the stent from a first end to a second end of the stent.
FIG. 25B illustrates a planar view of the stent of FIG. 25A rotated about the longitudinal axis.
FIG. 26 illustrates the as-cut configuration of a planar view of stent having unconnected adjacent windings, according to an embodiment of the present invention.
FIG. 27 illustrates the as-cut configuration of a planar view of stent having adjacent windings connected by a direct connector, according to an embodiment of the present invention.
FIG. 28 illustrates the as-cut configuration of a planar view of stent having adjacent windings connected by an indirect connector, according to an embodiment of the present invention.
FIG. 29A illustrates the expanded, deployed configuration of a planar view of the stent 100 shown in FIG. 27.
FIG. 29B illustrates a planar view of the stent 100 of FIG. 29A rotated about the longitudinal axis.
FIG. 30 illustrates a perspective or 3-dimensional (3D) view of the stent 100 shown in FIG. 29A and FIG. 29B.
FIG. 31 illustrates the stent 100 shown in FIG. 29B without the inner tube.
FIG. 32A illustrates the expanded, deployed configuration of a planar view of the stent 100 shown in FIG. 28.
FIG. 32B illustrates a planar view of the stent 100 of FIG. 32A rotated about the longitudinal axis.
FIG. 33 illustrates a perspective or 3D view of the stent 100 shown in FIG. 32A and FIG. 32B.
FIG. 34 illustrates the stent 100 shown in FIG. 32B without the inner tube.

### DETAILED DESCRIPTION OF THE INVENTION

In one aspect of the invention, the stent of the invention comprises a plurality of radiopaque markers mounted on the stent, in order to enhance X-ray visibility of the stent. Conventional stent designs offset radiographic markers by 180 degrees which disadvantageously results in the frontal or proximal marker blocking the distal marker (behind the proximal marker) when viewed head on. Further, conventional stent designs having two radiographic markers offset by 180 degrees result in only the frontal marker surface shape being visible when viewed head on and the side-view shape being visible from the side of the markers. In contrast, by using markers having multiple profiles or shapes and positioning those markers on the stent to be offset by an angle of less than 180 degrees, one of the radiographic markers will have a distinct shape or profile as compared to the other marker when viewed from any selected direction. In one embodiment, the plurality of radiopaque markers may be offset relative to another. The radiopaque markers may be offset relative to another by an angle less than 180 degrees such that the offset radiopaque markers may be advantageously observed, e.g., head on, during angiographic and/or radiographic imaging to enable improved stent navigation and placement within a vessel. Two or more markers may be provided at each end of the stent, where these markers (at the same end of the stent) are offset relative each other. The radiopaque markers at each end may be offset relative to a marker on the same end by 90 degrees, an angle more than 90 degrees, or an angle less than 90 degrees. For example, when two markers are positioned at each end of the stent, the two markers on that same end may be offset relative the other by about 90 degrees (±5 degrees). In another embodiment, when three markers are positioned at each end of the stent, the three markers on the same end may be offset relative the other by about 120 degrees (±5 degrees). Other angles less than 180 degree are also within the scope of the invention and it is also envisioned that the number of markers and/or the offset angle at each end of the stent may be the same or different. The shape or profile of the marker will be distinctive depending on the angle of observation. For example, when two hockey-puck shaped markers on the same end are offset by 90 degrees, the circular surface area of one marker will be visible when the first marker is viewed head on and the rectangular cross section of second marker will be visible.

The shape of the radiopaque markers may be a defined shape such as for example, circular, square, rectangle, triangle, oval or a defined irregular shape. Alternatively or in addition, the radiopaque markers may have shapes that may be identical or different but are offset to allow head-on differentiation of the markers. Further, the number of markers at opposite ends of the stent may be the same of or different. Exemplary radiopaque materials include platinum, tantalum, iridium, gold, among others, or alloys thereof. The radiopaque markers may be made of the same or different radiopaque materials. The radiopaque markers may be attached or mounted to the stent by swaging, pressing, welding, chemical bonding and other methods known in the art, for example. The radiopaque markers may be sized to have a diameter of 60-300 microns, and a thickness similar or the same as the thickness of the struts of the stent, such as 60-100 microns. Further, the radiopaque markers at each end of the stent may be mounted on a loop or a strut of the stent, such as on a loop or a strut of the end rings. Alternatively or in addition, the offset radiopaque markers may be mounted on any portion of the stent, and may not be limited to ends of the stent. It should be understood that features of radiopaque markers described herein, including the positioning of the markers contributing to the enhanced X-ray visibility of the stent, may be incorporated in any stent design or appropriate intraluminal endovascular device (*e.g.,* a stent, graft or stent-graft device).

This invention further relates to stents, in particular, the stent of the invention improves on existing stents by providing an advantageously designed serpentine or undulating pattern of struts connected to loops which provides for a reduced crimped profile and/or an enlarged expanded profile, as well as an optimal stress/strain distribution along the stent. The stent may be longitudinally flexible and radially rigid, where longitudinal flexibility is defined as the ability of the stent to flex about an axis of the stent which extends in a lengthwise direction of the stent. The loops are defined as portions of the serpentine pattern having about a 180 degree turn (*i.e.,* a U-turn) in the as-cut configuration of the stent, while the struts are portions of the serpentine pattern which have less than a 180 degree turn. Each end of the loop is connected to an end of a strut, such that each loop is connected to a pair of struts to form one undulation of the serpentine or undulating pattern.

The features of the invention, individually or in combination, advantageously provide for a stent having an increased expanded outside diameter in the expanded, deployed configuration and/or a reduced compressed outside diameter compared to conventional stents.

In particular, in one embodiment, the serpentine or undulating pattern of the stent is helically oriented in order to advantageously limit or avoid interference between adjacent loops in a crimped configuration of the stent. Adjacent loops are advantageously axially offset with respect to an axis perpendicular to the lengthwise direction of the stent to form a staggered pattern. The staggered pattern of the helical arrangement may be a uniform stagger, such that adjacent loops in the helical direction do not have a scalloped edge or profile. Instead of alignment of adjacent loops in the helical direction, the staggered pattern advantageously allows for loops to be positioned in alignment with an adjacent strut in the helical direction of the stent, which thereby avoids some of the interference between adjacent loops in the crimped configuration. The loops may be positioned in alignment with an end of the adjacent strut. Because loops have a large turn radius, the crimped diameter of the loops is still limited and the loops are the largest part of the stent in the crimped configuration of the stent.

A strut may have a length different than the remaining struts. In one embodiment, the loops of the undulating pattern may be connected to two struts of varying lengths, such that the undulating pattern of the stent comprises alternating long and short struts. This arrangement of varying the strut lengths also advantageously limits or avoids the interference between adjacent loops in the crimped configuration of the stent. An alternating pattern of long and short struts contributes to the reduced crimped profile of the stent by advantageously generating a staggered or offset pattern of adjacent loops with respect to a transverse axis, namely, an axis perpendicular to the lengthwise direction of the stent. The staggered pattern of adjacent loops advantageously avoids the interference between adjacent loops in the crimped configuration.

In addition to reducing the crimped profile, the arrangement of varying the strut lengths further provides the advantage of allowing for an enlarged expanded profile. Longer strut lengths are capable of expanding to a larger diameter when deployed than are shorter struts. By providing a stent having an alternating arrangement of long and short struts, where each loop is connected to one long strut and one short strut, the stent of the invention may advantageously expand to an enlarged expanded profile (due to the long strut) and compress to a reduced crimped profile (due, at least in part, to the short strut contributing to the staggered pattern). Further, the alternating arrangement of long and short struts, also advantageously contributes or enhances the flexibility of the stent, as well as the scaffolding and vessel wall coverage.

The arrangement of varying strut lengths may depend on the particular application of the stent and may be varied in a random or in a repeating periodic pattern. For example, one, some or all pairs of struts may comprise two struts of varied lengths (e.g., one long strut and one short strut). Where one or some of the pairs of struts comprise struts of varied lengths, the remaining pairs of struts may comprise two struts of the same length. Further, for example, the lengths of the struts may vary between different pairs of struts, such that, for example, a long strut of one pair may have a different length than a long strut of another pair, and/or a short strut of one pair may have a different length than a short strut of another pair. The strut lengths in the end rings may be varied in a same or different pattern than the strut lengths of the main stent component. In one embodiment, the struts of the end rings may not have variable lengths (i.e., may have the same lengths), while at least one strut of the main stent component may have a varied length. Alternatively, at least one strut of the end rings may have a variable length, while the strut of the main stent component may have the same lengths.

A strut of the present stent may have one or more bent sections, *e.g*., first and second bent sections in opposite curvature extending, from each end of the strut, toward an intersection point of the strut (*e.g*., at a mid-section of the strut). In one embodiment, the struts are bent, curved or arched, such that the struts are not straight or linear from one end of the strut to the other end, particularly, in the crimped configuration. In a pair of struts connected to a loop, the curvature of the bent section of the strut nearest the loop may bend inward (*e.g*., concave) toward the opposing strut of the pair, while the curvature of the bent section of the strut furthest from the loop may bend outward (*e.g*., convex) away from the opposing strut of the pair, such that the strut has a concave bent section and a convex bent section. The bent design of the strut may be referred to as a bent structure where the end of the strut connected to an end of a loop bends inward toward a center of the loop and then outward away from the center of the loop. This bent pattern of the struts exists and is maintained in the crimped, unexpanded, configuration as well as in the deployed, expanded, configuration of the stent, and during the configuration change, such that the bent sections do not substantially straighten. The bent pattern in a strut creates a space or a hollow section for an adjacent loop to fit therein in a nestled or nested arrangement as the stent is compressed, which in turn advantageously allows tighter packing or compressing of the stent in the crimped configuration. The bent strut pattern allows for the loops to nest into the bent section (*e.g*., the concave portion) of struts which are adjacent or opposite the loops in the helical direction when the stent is compressed into the crimped profile, thereby advantageously providing a reduced crimped profile. A stent of the present invention having at least one bent strut in the crimped configuration will advantageously have a compressed diameter smaller than a compressed diameter of a conventional stent having a strut which is substantially straight or substantially straightens during or when compressed because a straight or substantially straight strut does not allow for the advantageous nested arrangement of the present invention.

The number and/or arrangement of the bent struts may depend on the particular application of the stent, where the number of bent struts is inversely proportional to the crimped, delivery, diameter of the stent. A stent according of the invention, in the compressed and/or expanded configuration(s), may also have any combination of non-bent or straight/linear struts, bent struts for the long and/or the short struts, struts having different lengths, and struts having same or similar lengths some of which may be straight/linear struts while other struts are bent. Further, a stent may have such different strut lengths in a random pattern or repeating uniform pattern. In one embodiment, all of the struts of the main stent component may be bent struts. In another embodiment, the main stent component may have a mixed strut design, such that some of the struts are bent struts and some of the struts are linear struts. In yet another embodiment, the main stent component does not comprise a bent strut. In this embodiment, first and second end rings, connected to the main stent component, may comprise bent struts.

In the compressed configuration of the stent, at least some struts are bent while others may not be bent (*i.e*., are straight) in a uniform or random pattern. For example, alternating struts are bent, where, for example, only the long struts are bent. In another embodiment, the struts near the ends of the stent may not be bent while the remaining struts may be bent, or *vice versa.* In an embodiment where opposing struts of a pair of struts (*i.e*., two consecutive struts) each have one or more bends, the bends of the opposing struts may be out-of-phase (*e.g*., mirror image), but need not be in other embodiments. In another embodiment, substantially all struts of the stent include one or more bent sections, where no (or substantially no) struts are straight, in at least the compressed configuration of the stent. "Substantially all struts" may be defined as about 75% or more of the struts of the stent.

The bent strut design, in combination with the staggered pattern of adjacent loops in the helical direction, assists the loops to be positioned in alignment with an end of an adjacent strut in the helical direction such that, as the stent is compressed to the crimped profile, the loops are positioned to advantageously align with and nestle in the (*e.g*., concave) bent section of the adjacent strut (in the helical direction) which bends inward toward the adjacent loop. The stent of the invention may include the bent strut design and the staggered pattern of adjacent loops, individually or in combination, to assist in achieving a lower crimped profile of the stent. It should be noted that the staggered pattern of adjacent loops may be provided in a helical or spiral stent design or in a ring stent design having a series of separate, individual rings. Because the loop (which is the largest part of the stent pattern due to its turn radius) advantageously nestles in the (*e.g*., concave) bent section of an adjacent strut, the crimped profile of the stent may be further reduced, where interference between adjacent loops are avoided and interference between a loop and an adjacent strut is reduced. Thus, while the staggered pattern of adjacent loops reduces the crimped profile by minimizing the interference produced by adjacent loops, the bent strut design further reduces the crimped profile by additionally minimizing interference between a loop and an adjacent strut. Interference between a loop and an adjacent strut is reduced because the bent section of the strut at least partially envelopes the adjacent loop, for example, enveloping one end of the loop to about a mid-section of the loop. The bent strut design allows the bent section of the strut (opposite an adjacent loop in the helical direction) to have a complementary shape to the adjacent loop such that there is a complementary or lock-and-key fit between a loop and the opposing bent section of the adjacent strut. Thus, the crimped profile of the present invention is further reduced because the bent section advantageously conforms to and allows nestling of the adjacent loop. As such, the bent sections of the struts advantageously maintain the bent strut pattern (and are not straight or straighten) in the crimped configuration of the stent.

Optimal stress/strain distribution along the stent may be further advantageously achieved by redistributing the stress/strain forces imparted on the stent to prevent permanent deformation of the stent, and enable the stent to fully expand or fully compress. The stress/strain imparted on the stent may be advantageously redistributed by varying the relative strength or flexibility of different portions of the stent, such as by redistributing the stress/strain forces away from the loops. For example, the amount of material used to form different portions of the stent can be varied to change the portions' relative strength or flexibility. This variation in strength or flexibility of stent portions can be accomplished by increasing the thickness or width of the loop portions to increase the strength of these portions relative to the strut portions, thereby redistributing stress/strain forces away from the loop portions. Alternatively or in addition, the strut width is also gradually decreased from both ends towards the strut's mid-section to further redistribute stress/strain forces away from the loop portions and toward the mid-section of the strut portion of the stent.

In one embodiment, the stent may comprise a polymer material. The polymer material may be electrospun onto the stent. The polymer material may interconnect at least two of the plurality of windings of the stent. The polymer material may be a biodegradable polymer, or may be another polymer. In one embodiment, the polymer material may further comprise a drug embedded therein.

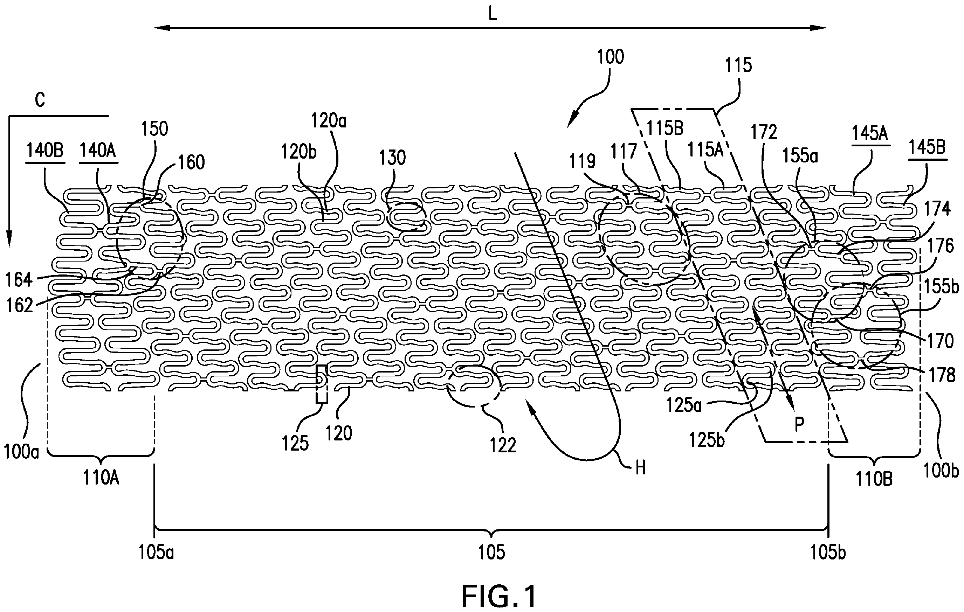

FIG. 1 illustrates a stent 100 in the as-cut configuration according to an embodiment of the invention which, for illustration purposes only, is shown in a longitudinally opened and flattened view. In use, the stent 100 has a tubular shape and may be manufactured from an extruded tube, or a flat sheet which is rolled into the tubular shape. The desired stent design or pattern may be laser cut onto the extruded tube, or may be laser cut or chemically etched onto the flat sheet which is then rolled. The stent 100 of FIG. 1 is shown in the as-cut configuration, which is a neutral profile of the stent 100 when the stent 100 has been formed (*i.e*., manufactured), but not yet crimped to the delivery diameter and not yet expanded to the implanted deployed diameter.

The stent 100 is a tubular structure having a main stent component 105 extending from a first end 105a to a second end 105b along a lengthwise direction L of the stent 100. The main stent component 105 is arranged to have a helical orientation along a helical direction H of the stent 100. In one embodiment, as shown in FIG. 1, the continuous tubular structure includes a first end ring 110A and a second end ring 110B positioned to extend from the first end 105a and the second end 105b, respectively. The first and second end rings 110A-B extend in a circumferential direction C around a circumference of the stent 100 such that the first and second end rings 110A-B, at lengthwise ends 100a-b of the stent 100, are oriented approximately at a right or 90° angle to the lengthwise direction L to form a right-angled cylinder. Approximately at a right angle with respect to the first and second end rings 110A-B is defined as oriented at any angle closer to 90° than the helical orientation of the main stent component 105. In another embodiment (not shown), the stent 100 is the main stent component 105 without the first and second end rings 110A-B, such that lengthwise ends 100a-b of the stent 100 are the first and second ends 105a-b and do not form a right-angled cylinder relative to the lengthwise direction of the stent.

The main stent component 105 includes a plurality of windings 115. The windings 115 are continuously oriented in the helical direction H between the first and second ends 105a-b, such that the windings 115 are oriented at an oblique angle to the lengthwise direction L of the stent 100. Each winding 115 may include one or more bands. In the exemplary embodiment shown in FIG. 1, each winding 115 includes a first band 115A and a second band 115B which are interconnected to one another, thereby forming two interconnected bands 115A-B. The first band 115A and the second band 115B are interconnected to one another to form cells 117 there-between and are oriented in the helical direction H of the stent 100, such that the cells 117 form a helix of cells between the first end 105a and the second end 105b. The area enclosed by the cells 117 are open spaces or gaps between the interconnected bands 115A-B.

The first and second bands 115A-B each has a serpentine or undulating pattern and extend generally parallel to each other in the helical direction H. The undulations of the first and second bands 115A-B comprise struts 120 connected to one another by a pattern of loops 125, referred to as peaks 125a and valleys 125b.

The loops 125 are portions of the undulating pattern having a turn of about 180 degrees (i.e., a U-turn), while the struts 120 do not. One or more of the struts 120 may have one or more bends (e.g., one or more bent sections) having a turn of less than 180 degrees. Some of the struts 120 may have no bends (i.e., may be straight or linear members). Each end of a loop 125 is coupled to an end of a strut 120, thereby forming a pair of struts 122 connected to a loop 125. The pair of struts 122 are two struts connected to a common loop and which are adjacent to each other in the helical direction H within a winding 115.

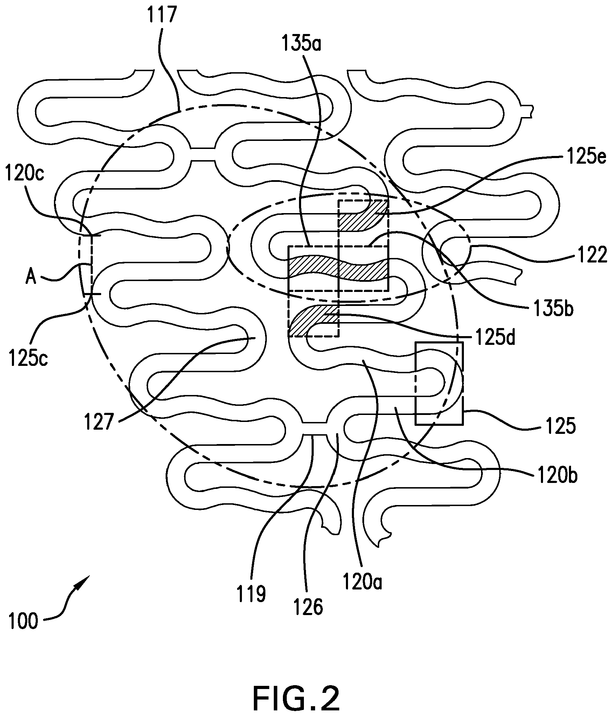

The number and/or location of the struts 120 having a bent strut design may vary depending on a particular application. A stent 100 having more struts 120 which are bent results in a greater reduced crimped profile because bent struts of the invention allow for tighter packing in the helical direction H between adjacent loops 125 and struts 120 (at, *e.g*., the first and second bent sections 135a-b shown in FIGs. 2-3) and allow for avoidance of interference (or contact) between adjacent loops 125. In an embodiment having a mixed bent/linear strut design, the struts 120 which are not bent (*i.e*., linear) may be at or near the lengthwise ends 100a-b of the stent 100, such as, for example, in the first and second end rings 110A-B and/or in the windings 115 at the first and second ends 105a-b of the main stent component 105. Alternatively or in addition, the struts 120 which are not bent (*i.e*., linear) may be randomly or uniformly located throughout the stent 100.

The loops 125 form the serpentine pattern such that the peaks 125a and valleys 125b are arranged in an alternating pattern in the helical direction H of each of the first and second bands 115A-B. The peaks 125a are the loops 125 which curve toward the opposing interconnected band 115A-B of a winding 115 and thus are the outer loops 125a of the cell 117. The valleys 125b are the loops 125 which curve away from the opposing interconnected band 115A-B of a winding 115 and thus are the inner loops 125b of the cell 117. The valleys 125b are closer, along the lengthwise direction L, to the center of a cell 117 enclosed by the interconnected bands 115A-B than are the peaks 125a. The center of the cell 117 are points along a point axis P extending between two points of interconnection which interconnect the first and second bands 115A-B to define a cell 117. The number, type and/or location of interconnections in each winding 115 of the stent may be at regular or uniform intervals (*e.g*., every third pair of helically adjacent valleys 125b shown in FIG. 1) or may be at random intervals, and may depend on a particular application (*e.g*., coronary or peripheral vessel applications). Some or all of the interconnections may extend substantially lengthwise along the lengthwise direction L of the stent 100, but may extend or be oriented along other directions (*e.g*., circumferentially and/or helically).

As shown in FIG. 1, the two interconnected bands 115A-B in a winding 115 may be substantially out-of-phase with each other such that, along the lengthwise direction L, peaks 125a of the first and second bands 115A-B are substantially aligned with or face each other, and valleys 125b of the first and second bands 115A-B are substantially aligned with or face each other. In this out-of-phase embodiment, a distance (*i.e*, open space or gap) spanning a length of the cell 117 (along the lengthwise direction L) between aligned valleys 125b of the first and second bands 115A-B in a winding 115 is smaller than a distance spanning the length of the cell 117 between aligned peaks 125a of the first and second bands 115A-B in the winding 115. In another embodiment (not shown), the two interconnected bands 115A-B in a winding 115 may be substantially in-phase with each other such that, along the lengthwise direction L, peaks 125a of the first band 115A may be substantially aligned with or face valleys 125b of the second bands 115B. In another embodiment, the stent 100 may have mixed phase interconnected bands 115A-B in a winding 115, such that some or at least one interconnected band 115A-B in a winding 115 may be substantially out-of-phase with each other, while the remaining interconnected bands 115A-B in remaining windings 115 may be substantially in-phase with each other.

As shown in FIG. 1, a first band 115A in a winding 115 and a second band 115B in an adjacent winding 115 may be substantially in-phase with each other such that, along the lengthwise direction L, peaks 125a of the first band 115A may be substantially aligned with or face valleys 125b of the second bands 115B of the adjacent winding 115. In another embodiment (not shown), the first band 115A in the winding 115 and the second band 115B in an adjacent winding 115 may be substantially out-of-phase with each other such that, along the lengthwise direction L, peaks 125a of the first band 115A are substantially aligned with or face peaks 125a of the second band 115B of the adjacent winding 115, and valleys 125b of the first band 115A are substantially aligned with or face valleys 125b of the second band 115B of the adjacent winding 115. In yet another embodiment, the stent 100 may have mixed phase adjacent windings 115, such that some or at least one first band 115A in a winding 115 and a second band 115B in an adjacent winding 115 may be substantially in-phase with each other, while the adjacent first and second bands 115A-B in the remaining adjacent windings 115 may be substantially out-of-phase with each other.

The loops 125 are axially offset with respect to a perpendicular axis to the lengthwise direction L. In the embodiment shown in FIG. 1, an apex (outer tip) of a loop 125 is aligned with an end of an adjacent or opposing strut 120 at the end connected to the adjacent loop 125. The staggered pattern of alignment A of adjacent loops in the helical direction H is identified in FIG. 2, where the apex 125c of a loop 125 is aligned with an end 120c of an adjacent strut 120 in the helical direction H. Other staggered patterns of alignment may be incorporated into the stent 100 of the present invention, where the plurality of windings 115 of the stent 100 may have a consistent staggered pattern of alignment, or may have varied staggered patterns of alignment, for example, in different windings 115 and/or within the same winding 115.

In FIG. 1, the pair of struts 122 comprise two struts 120 having different lengths connected to a common loop 125. For example, the pair of struts 122 comprise a one long strut 120a and one short strut 120b, where a length of the long strut 120a is longer than a length of the short strut 120b. In FIG. 1, the lengths of the long strut 120a and the short strut 120b are sized such that adjacent loops 125 in the helical direction H are axially offset with respect to a perpendicular axis to the lengthwise direction L of the stent 100 to form the staggered pattern of alignment A of adjacent loops, where a loop 125 (*e.g*., at its apex 125c) is positioned to align with an end 120c of an adjacent strut 120.

The stent 100 comprises at least one strut 120 or one pair of struts 122 having a length different than the remaining struts 120 or pairs of struts 122 of the stent 100. Alternatively, in another embodiment (not shown), the stent of the invention may comprise struts of all the same length. In the embodiment shown in FIG. 1, the long and short struts 120a-b are arranged in an alternating arrangement about the helical direction H. The strut lengths may be similarly varied in the first and second end rings 110A-B. In the embodiment shown in FIG. 1, at least one strut 120 of the first and second end rings 110A-B has a length different than the remaining struts 120 of the first and second end rings 110A-B, and at least one strut 120 of the main stent component 105 has a length different than the remaining struts 120 in the main stent component 105.

The stent 100 of FIG. 1 includes at least one strut 120 having a bent strut design facilitating the reduced compressed profile of the stent 100. The bent strut design or pattern comprises at least one strut having one or a plurality of bends along the strut length. Where the strut length includes a plurality of bends, at least one strut 120 includes at least two bends which face opposite each other such that the pair of struts 122 connected by a loop 125 form a bent pattern, structure or shape 130, as shown in FIG. 1. In the crimped configuration of the stent 100, the bent strut design of at least one strut 120 having at least two opposing bends allows for adjacent loops 125 (*e.g.,* bottom and top loops 125d-e of FIG. 2) in the helical direction H to nestle in the two opposing bends, respectively, thereby providing a reduced compressed diameter. Alternatively or in addition, at least one strut 120 of the stent 100 may form the bent shape 130 along a length of the strut 120, such that, in the crimped configuration of the stent 100, at least one adjacent loop 125 (*e.g.,* a top or a bottom loop 125) in the helical direction H nestles in the opposing bend of the strut 120, thereby providing a reduced compressed diameter. In an embodiment (not shown), at least one strut 120 comprises a bent strut design where the strut 120 has one curve in a first section along the strut 120 length, while the remaining length of the strut 120 is linear or straight (*i.e*., absent a curve), such that an adjacent loop 125 (*e.g.,* a top or bottom loop 125) in the helical direction H may nestle in the bent first section of the strut 120, in the crimped configuration of the stent 100. The one or more bends in at least one strut 120 of the stent 100 (in any and all embodiments) are maintained in the crimped profile of the stent 100, such that when the stent is compressed or during compression, the one or more bends do not substantially straighten, and thereby providing the nestled arrangement and reduced crimped profile of the stent 100.

FIG. 2 illustrates an enlarged view of an enclosed cell 117 of the main stent structure 105 of the stent 100 of FIG. 1, where the bent strut design is more clearly identifiable. The bent strut design of at least one strut 120 includes a first bent section 135a and a second bent section 135b extending in an opposite direction from each end 120c of the strut 120 toward a mid-section of the strut 120, where each end 120c is the portion of the strut 120 contiguous with a loop 125. One of the bent sections 135a-b is preferably convex while the other bent section 135a-b is concave. For example, in a pair of struts 122 having at least one strut 120 with the bent strut design, the first bent section 135a of the strut 120 extends from the end of the loop 125 coupled to the pair of struts 122 and curves inwards (*e.g*., concave) toward an opposing strut 120 of the pair 122 to form the bent structure 130. The second bent section 135b of the strut 120 extends from the mid-section of the strut 120 to an end of an adjacent loop 125 in a substantially the lengthwise direction L, where the second bent section 135b bends outwards away (*e.g*., convex) from the opposing strut 120 of the pair 122 to form the bent structure 130. The bent strut design (*e.g*., the first and second bent sections 135a-b) creates a space, hollow or area/volume for an adjacent loop 125 (*e.g.*, 125d-e) in the helical direction H to fit in the space created (*i.e*., nestle in the bent sections 135a-b) when the stent 100 is compressed or is in the crimped, delivery configuration. For example, the nestled arrangement is such that, as the stent is compressed, the first bent section 135a and the adjacent loop 125d below the first bent section 135a (in the helical direction H) move toward each other so that the below adjacent loop 125d nestles into the first bent section 135a in the crimped, delivery configuration. Similarly, as the stent is compressed, the second bent section 135b and the adjacent loop 125e above the second bent section 135b (in the helical direction H) moves toward each other so that, in the crimped delivery configuration, the above adjacent loop 125e nestles into the second bent section 135b. In the as-cut configuration of the stent 100, shown in FIG. 2, the first and second bent section 135a-b are aligned (along a perpendicular axis to the lengthwise direction L) with, but not nestled with, adjacent bottom and top loops 125d-e, respectively. Similarly, in the expanded, implanted or deployed, configuration of the stent 100, the first and second bent section 135a-b may be aligned (along a perpendicular axis to the lengthwise direction L) with, but not nestled with, adjacent bottom and top loops 125d-e, respectively.

The area of the first and second bent sections 135a-b which are aligned and configured to nestle with respective bottom and top adjacent loops 125d-e in the crimped profile of the stent 100 are illustrated in FIG. 2 by the cross-hatching. As illustrated in FIG. 2, the first bent section 135a, at an end of the strut 120, is aligned with the apex 125c of the below adjacent loop 125d in the helical direction H, while the first bent section 135a, near the mid-section of the strut 120, is aligned with an end of the below adjacent loop 125d, such that the first bent section 135a aligns with an adjacent (*e.g*., bottom) loop 125d. As such, adjacent loops 125 in the helical direction H are axially offset, *i.e*., staggered, with respect to a perpendicular axis to the lengthwise direction L. The staggered pattern of alignment A of adjacent loops in the helical direction H are staggered such that overlap between adjacent loops 125 is avoided or limited when the stent 100 is compressed to the crimped profile, thereby allowing for a reduced compressed diameter. Further, the staggered pattern of alignment A of adjacent loops is such that an adjacent loop 125 is positioned to align (along a perpendicular axis to the lengthwise direction L) with the respective, opposing, bent section 135a-b of the adjacent strut 120 where, as the stent 100 is compressed to the crimped profile, the loop 125 nestles in the respective, opposing, bent sections 135a-b, thereby further reducing the compressed diameter. In one embodiment, the nestling arrangement may be such that at least one loop 125 has a substantially complementary fit with an opposing bent section 135a-b in the helical direction H. The nestling arrangement may be such that at least one loop 125 is nestled to contact (or be in near-contact with) the opposing bent sections 135a-b when the stent is in the crimped configuration.

In any and all embodiments of the present invention, the first and/or second bent sections 135a-b of at least one strut 120 must be bent (*e.g.*, either maintain or become more bent), as the stent 100 is compressed or when the stent 100 is in the compressed configuration, so that at least one adjacent loop 125 in the helical direction H is configured to nestle in the opposing or respective first and/or second bent section 135a-b, thereby reducing the compressed diameter of the stent 100. In one embodiment, the first and second bent sections 135a-b are bent (*i.e.*, do not straighten) in the crimped, as-cut, and expanded diameters of the stent 100. In another embodiment, in order to provide for an enlarged expanded diameter, the first and/or second bent sections 135a-b may substantially straighten in the expanded, deployed profile of the stent 100 such that the first and/or second bent sections 135a-b become more straight compared to the more bent crimped or as-cut profiles or may become entirely straight. The struts 120 may substantially straighten in the expanded profile and/or may become more bent in the crimped profile because the struts 120 are flexible.

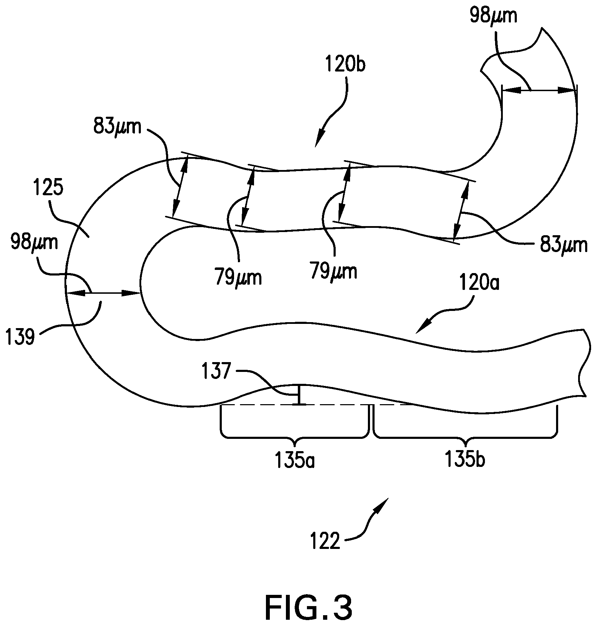

The amount of curvature of the first and second bent sections 135a-b may depend on a particular application, where a higher bend in the first and second bent sections 135a-b may result in a greater reduced crimped profile, and may therefore be preferable in coronary applications in contrast to peripheral applications. The amount of curvature may be defined as the curvature angle of each of the first and second bent sections 135a-b, respectively. The curvature angle is created by the bend of the strut 120 at the first and second bent sections 135a-b and provides a gap, space or hollow such that the adjacent loop may nestle therein in the crimped configuration of the stent 100. The curvature angle provides a maximum height 137 (FIG. 3) of the gap, space or hollow created by the one or more bends of the strut 120. The curvature angle is less than 90 degrees but greater than zero degrees, and may be greater than 35 degrees. The amount of curvature of the first and second bent sections 135a-b of a strut 120 may be the same or different, and the amount of curvature in different struts 120 may be the same or different. In one embodiment, the height 137 (FIG. 3) of the curvature (*i.e*., the maximum height 137 of the gap or space created by the bend of the first and/or second bent sections 135a-b) may be greater than 0 microns but less than about 150 microns, and may preferably be at least 30 microns. Alternatively or in addition, the height 137 of the curvature may be substantially the same as or equal to the width of the loop 125 (for example, at the width of the loop 125 at the portion which is configured to nestle within the space created by the bend of the bent sections 135a-b). Alternatively, the height 137 of the curvature may be about half the width 139 (FIG. 3) of the loop 125.

Similarly, the number and/or location of the struts 120 having a bent strut design may vary depending on a particular application. The stent 100 includes at least one strut 100 having the bent strut design of at least one of the first and second bent sections 135a-b. In the embodiment shown in FIGs. 1 and 2, for example, a mixed bent/linear strut design is illustrated in an alternating pattern such that each pair of struts 122 includes the long strut 120a having the first and second bent sections 135a-b in opposite orientation, and the short strut 120b which is linear. In the embodiment shown in FIGs. 1-2, the main stent component 105 includes the alternating pattern of bent long struts 120a and linear short struts 120b, while the struts 120 of the end rings 110A-B are linear and not bent. However, in other embodiments and as required for a particular application, the end rings 110A-B may include at least one strut 120 having a bent strut design. Further, in other embodiments with the alternating pattern, the short strut 120b may comprise the bent strut design and the long strut 120a may be linear, or both struts 120 of the pair 122 (*e.g*., the long and short struts 120a-b) may comprise the bent strut design.

In the embodiment shown in FIGs. 1-2, the first and second bands 115A-B are connected to each other to form the cells 117 by at least one link 119. The links 119 are connectors or cross-struts which extend in the lengthwise direction L of the stent 100 and/or may extend in a diagonal direction (not shown) to form the two interconnected bands 115A-B and enclose a cell 117. The links 119 extend in a gap between the first and second bands 115A-B, thereby closing or forming the cells 117. The links 119 may be flexible connectors, thereby enabling the stent 100 to conform to the curvature of a vessel anatomy. In the embodiment shown in FIG. 1, the links 119 are straight or linear connectors absent a bend. Alternatively, in another embodiment (not shown), the links 119 may have one or more loops or bends, or some links 119 may be linear connectors while other links 119 in the stent 100 may have loops or bends. Alternatively to links 119, the first and second bands 115A-B may be connected directly to each other to form the cells 117 absent links 119. In another embodiment, the stent 100 may include a combination of links 119 and direct connections for interconnecting the first and second bands 115A-B in a winding 115. Other interconnections and direct connections may be achieved and are within the scope of the present invention, such as, for example by weld, adhesive, metal connectors, a polymer material, or any form of physical joining or other securement, interlock or connection means. The loops 125 at which the interconnections are positioned may be referred to as attachment loops 126 and the loops 125 at which no interconnection is positioned may be referred to as free loops 127, as shown in FIG. 2.

The number, type, and/or location (*e.g*., interval or placement) of interconnections (*e.g*., links 119 and/or direct connections) may depend on a particular application (*e.g.,* coronary or peripheral vessel applications), where the interconnections determine the size and shape of the cell 117. In the exemplary embodiment shown in FIGs. 1-2, the first and second bands 115A-B in a winding 115 are connected by links 119 at every sixth loop 125 (or every third valley 125b), for example. Accordingly, in the exemplary embodiment shown in FIGs. 1-2, each cell 117 is enclosed by two links 119 (between attachment loops 126), twelve struts 120, and ten free loops 127. Further, for example, as shown in FIG. 2, the attachment loops 126 are valleys 125b, such that the links 119 are positioned to connect the apex of a valley 125b of the first band 115A and the apex of a valley 125b of the second band 115B which are aligned along the lengthwise direction L. As such, in this embodiment, the links 119 are arranged to connect the first and second bands 115A-B at locations at which the gap or distance there-between is the smallest, which thus results in a shorter link 119 (compared to a link that may span the largest distance between the two interconnected bands 115A-B). In alternative embodiments, the number, interval and/or location of the interconnections (*i.e*., links 119 or direct connections) may differ from that illustrated in FIGs. 1-2.

In some embodiments, the links 119 may each have the same thickness relative to each other and/or to the first and second bands 115A-B or a portion thereof. Alternatively, the links 119 may have a different thickness, for example, a smaller thickness than the first and second bands 115A-B (or any portion thereof), or a different thickness from each other, as appropriate for a particular use. Links 119 having a narrower thickness provide for greater flexibility to the stent than links 119 having wider thickness, while links 119 with wider thickness provide greater structural integrity and rigidity to the stent. The links 119 may have a uniform thickness along the link length or a variable thickness. Further, in some embodiments the plurality of links 119 have the same lengths or varying lengths at uniform or random intervals. In one embodiment, for a coronary stent, links 119 may vary in length from 0.05 mm to 0.15 mm and may vary in thickness from 0.03 mm to 0.07 mm. In another embodiment, for a peripheral stent, links 119 may vary in length from 0.5 mm to 1.0 mm and may vary in thickness from 0.05 mm to 0.1 mm. The lengths and thickness of the links 119 may depend on the stent application (*e.g.,* coronary or peripheral), type of deployment (*e.g.,* balloon expandable or self-expandable) and/or stent target diameter.

Similarly, the lengths and thickness of the struts 120 may depend on the stent application (*e.g.,* coronary or peripheral), type of deployment (*e.g.,* balloon expandable or self-expandable) and/or stent target diameter. All or some of the struts 120 may have a same thickness and/or length relative to each other, or a different thickness and/or length from each other. In one embodiment, for a coronary stent, struts 120 may vary in length from 0.5 mm to 1.5 mm and may vary in thickness from 0.04 mm to 0.1 mm. In another embodiment, for a peripheral stent, struts 120 may vary in length from 1.3 mm to 2.5 mm and may vary in thickness from 0.08 mm to 0.14 mm. In yet another embodiment, the strut thickness may be less than 0.065 mm for all stent sizes without the need to compensate by increasing the number of struts and/or links, thus minimizing the overall metal content of the stent and thereby advantageously providing high overall stent flexibility. Further, all or some of the struts 120 may have a single thickness from one end of the strut 120 to the other end, or all or some of the struts 120 may have more than one thickness along the length of the strut 120 from one end to the other end. The struts 120 (at any portion along the strut length) may have a same or different thickness than the loop 125.

FIG. 3 illustrates an enlarged view of a pair of struts 122 connected to a loop 125 of a stent 100 in the as-cut configuration according to another embodiment of the invention. In some embodiments of the invention, each strut 120 of the pair of struts 122 may have the same length, where each strut 120 has the bent strut design. In the one embodiment shown in FIG. 3, a long strut 120a and a short strut 120b of a pair of struts 122 each has the bent strut design of the first and second bent sections 135a-b, and are connected to each other by a loop 125. The bent strut design 135a-b of the long strut 120a is substantially a mirror image of (or out-of-phase with) the bent strut design 135a-b of the short strut 120b of the pair 122. In the embodiment having a pair of struts 122 with the bent strut design as in FIG. 3, the pair of struts 122 with the bent strut design may be in an alternating or other uniform arrangement with a pair of struts 122 with a linear strut design or may be arranged in a random pattern. However, without departing from the scope or spirit of the invention, the bent strut design may be in only one strut 120 of the pair 122 and/or the pair of struts 122 may each be the same length. The stent of the embodiment shown in FIG. 3 may include all or some of the features in any combination, as described above and/or in relation to the embodiment shown in FIGs. 1-2. FIG. 3 illustrates an embodiment where the struts 120a-b have varying width from one end to the other, and where the loops 125 have a greater width than any portion of the struts 120a-b in order to optimally redistribute the stress/strain concentrations imparted on the stent 100 away from the loops 125 and toward the struts 120. As shown in FIG. 3, the width 139 of the loop 125 is about 98 microns, while the width of the struts 120a-b vary from about 79 to 83 microns. The width of the struts 120a-b may gradually decrease or taper from both ends toward a mid-section of the strut to redistribute the stress/strain forces away from the loop 125 and toward the midsection of the strut 120a-b.

Referring back to FIG. 1, the first and second ends 105a-b of the main stent component 105 includes first and second end rings 110A-B at the lengthwise ends 100a-b of the stent 100. The first end ring 110A may comprise at least one first circumferential end band 140A and at least one second circumferential end band 140B interconnected in the lengthwise direction L to form two interconnected circumferential end bands 140A-B at a lengthwise end 100a of the stent 100. Similarly, the second end ring 110B comprises at least one first circumferential end band 145A and at least one second circumferential end band 145B interconnected in the lengthwise direction L to form two interconnected circumferential bands 145A-B at the other lengthwise end 100b of the stent 100. Both of the two interconnected circumferential end bands 140A-B, 145A-B are substantially similar to the first and second bands 115A-B of the windings 115, except each of the two interconnected circumferential end bands 140A-B, 145A-B are oriented around a circumference of the stent 100 in the circumferential direction C, and are not arranged in the helical direction H. The two interconnected circumferential end bands 140A-B, 145A-B in the circumferential direction C form first and second end rings 110A-B oriented approximately at a right cylinder (forming a right or 90° angle with respect) to the lengthwise direction L of the stent 100. The lengthwise ends 100a-b of the stent 100 (at the end rings 110A-B) may have a straight cross-sectional profile. When the lengthwise ends 100a-b of the stent 100 are not straight (*e.g*., due to a non-uniform staggered or offset pattern of adjacent loops in the circumferential direction C), the lengthwise ends 100a-b have a non-uniform (*e.g*., a scalloped edge), which may be a random or periodic pattern.

With respect to the common features, the end rings 110A-B comprise, in the same manner as discussed with respect to the main stent component 105, one or more of the following exemplary features: the undulating pattern of loops connected to a pair of struts (including the in-phase or out-of-phase orientations), the variable (or non-variable) strut lengths, the staggered or offset pattern of adjacent loops, the bent strut design (including, but not limited to, the alignment and nestling arrangement between a loop and a bent section of a strut), redistribution of the stress/strain forces (such as a variable strut width, and/or loops having a different width relative to the strut width), the cellular design (including the number and/or interval of placement of links and/or direct connections) and/or any other combination of features, embodiments or configurations, such as described with respect to the main stent component 105.

The first and second end rings 110A-B extend from the winding 115 adjacent thereto. The transition from the winding 115 of the main stent component 105 to the first and/or second end rings 110A-B may result in one or more cells that are referred to as transition cells. The transition cell may be different than other cells (*e.g*., cells 117) of the stent 100 in that a transition cell may be formed or enclosed by at least a portion of the undulating pattern (*i.e*., struts and/or loops) of the first and/or second band 115A-B and by at least a portion of the undulating pattern (*i.e*., struts and/or loops) of the first and/or second circumferential end band 140A-B, 145A-B. Thus, the transition cells are enclosed by both the main stent component 105 and the first and/or second end rings 110A-B, rather than only by the main stent component 105, or only by the first or second end rings 110A-B.

Further, the area enclosed by a transition cell may be different in size and/or shape than the area enclosed by cells 117 of the main stent component 105 and/or cells formed between the two interconnected circumferential end bands 140A-B, 145A-B. The one or more transition cells between the first end ring 110A and an adjacent winding 115, and the one or more transition cells between the second end ring 110B and an adjacent winding 115 may be the same or different, for example, with respect to number, size, shape, orientation, location, and/or type of interconnection (*e.g.*, links or direct connections). Further, in an embodiment having a plurality of transition cells between the first end ring 110A and an adjacent winding 115, the transition cells may be the same or different. Similarly, a plurality of transition cells between the second end ring 110B and an adjacent winding 115 may be the same or different.

FIG. 1 shows exemplary first, second and third transition cells 150, 155a, 155b, however, other transition cells having different sizes and/or configurations are within the scope of the present invention so long as the transition cell is formed between the main stent component 105 and the first or second end rings 110A-B. The transition between the main stent component 105 and the first or second end rings 110A-B may include any one or a combination of the exemplary first, second and third transition cells 150, 155a, 155b shown in FIG. 1 or other transition cells (not shown). The struts bordering the transition cells 150, 155a, 155b may have the same or variable lengths in order to enclose a desired area size and/or impart a desired flexibility or structural rigidity to the stent 100 at the transition between the main stent component 105 and the first or second end ring 110A-B, as is desired for a particular application. Some, all or none of the struts of the transition cells 150, 155a, 155b may include the bent strut design, the staggered or offset pattern of adjacent loops, and/or the redistribution of the stress/strain concentrations away from the loops (*e.g.*, by struts having variable widths and/or loops having a greater width relative to the struts).

The first transition cell 150 is formed between the first end ring 110A and an adjacent winding 115 of the main stent component 105. The first transition cell 150 is enclosed by a portion of the undulating pattern of the first band 115A, the second band 115B and the first circumferential end band 140A. The first transition cell 150 is enclosed by an interconnection 160 (*e.g.*, direct connection) between the first circumferential end band 140A (*e.g.,* at a strut thereof) and the first band 115A (*e.g.,* at an apex of a valley 125b thereof). The first transition cell 150 is also enclosed by an interconnection 162 (*e.g.,* a link 119) between the first band 115A (*e.g.,* at an apex of a valley 125b thereof) and the second band 115B (*e.g*., at an apex of a valley 125b thereof). Further, the first transition cell 150 is enclosed by an interconnection 164 (*e.g.,* a direct connection) between the second band 115B (*e.g*., at a strut 120 thereof) and the first circumferential end band 140A (*e.g.,* at an apex of a peak thereof). In the illustrative embodiment shown in FIG. 1, the first transition cell 150 is bordered by six struts 120 of the first band 115A connected by five free loops 125 of the first band 115A, one strut 120 of the second band 115B, and six struts of the first circumferential end band 140A connected by five loops of the first circumferential end band 140A.

The second transition cell 155a is substantially similar to the first transition cell 150, but is formed between the second end ring 110B and an adjacent winding 115 of the main stent component 105. The second transition cell 155a is enclosed by a portion of the undulating pattern of the first band 115A, the second band 115B and the first circumferential end band 145A. The second transition cell 155a is enclosed by an interconnection 170 (*e.g*., direct connection) between the first circumferential end band 145A (*e.g.,* at a strut thereof) and the second band 115B (*e.g.,* at an apex of a valley 125b thereof). The second transition cell 155a is also enclosed by an interconnection 172 (*e.g.,* a link 119) between the first band 115A (*e.g.,* at an apex of a valley 125b thereof) and the second band 115B (*e.g*., at an apex of a valley 125b thereof). Further, the second transition cell 155a is enclosed by an interconnection 174 (*e.g.,* a direct connection) between the first band 115A (*e.g.,* at a strut 120 thereof) and the first circumferential end band 145A (*e.g*., at an apex of a peak thereof). The second transition cell 155a is bordered by six struts 120 of the second band 115B connected by five free loops 125 of the second band 115B, one strut 120 of the first band 115A, and six struts of the first circumferential end band 145A connected by five loops of the first circumferential end band 145A.

The third transition cell 155b is formed between the second end ring 110B and an adjacent winding 115 of the main stent component 105. The third transition cell 155b is enclosed by a portion of the undulating pattern of the second band 115B, the first circumferential end band 145A and the second circumferential end band 145B. The third transition cell 155b is enclosed by an interconnection 176 (*e.g.,* a link) between the first circumferential end band 145A (*e.g.*, at an apex of a valley thereof) and the second circumferential end band 145B (*e.g*., at an apex of a valley thereof). The third transition cell 155b is also enclosed by an interconnection 178 (*e.g.,* a link) between the second band 115B (*e.g*., at an apex of a valley 125b thereof) and the second circumferential end band 145B (*e.g*., at an apex of a valley thereof). Further, the third transition cell 155b is enclosed by the interconnection 170 (*e.g.,* a direct connection) between the first circumferential end band 145A (*e.g.,* a strut thereof) and the second band 115B (*e.g.,* at an apex of a valley 125b thereof). The third transition cell 155b is bordered by six struts of the second circumferential end band 145B connected by five free loops of the second circumferential end band 145B, four struts 120 of the second band 115B connected by three loops 125, and three struts of the first circumferential end band 145A connected by two loops of the first circumferential end band 145A. In another embodiment (not shown), a transition cell similar to the third transition cell 155b may be similarly formed between the first end ring 110A and an adjacent winding 115, where this transition cell may be enclosed by the first band 115A, the first circumferential end band 140A and the second circumferential end band 140B.

The stent according to embodiments disclosed herein may be useful for coronary or non-coronary applications such as a peripheral stent, a brain stent or other non-coronary applications. For coronary use, the stent may vary in length from 6-60 mm, have an expanded, deployed outside diameter of 1.5-6.0 mm, and a compressed, delivery outside diameter of 0.7-1.3 mm. For non-coronary use (*e.g*., peripheral applications), the stent may vary in length from 20-250 mm, have an expanded, deployed diameter of 3-8 mm, and a compressed, delivery diameter of 0.7-2.0 mm. Further for coronary use, the stent may have a cell design with fewer interconnections (*e.g*., links or direct connections) and thus larger cells in order to provide a stent having cells sufficiently large for increased side branch access which is advantageous for use in the tortuous coronary vessels having multiple side branches. The cells of the stent may be the same or similar size along substantially the entire length of the stent or at least along the main body of the stent (excluding the ends) in order to provide similar side branch access and support throughout. In the illustrative embodiment shown in FIG. 1, each winding 115 of the stent 100 has three interconnections and nine peaks or crowns 125a. However, any other number of interconnections or peaks may be selected for a particular application and desired stent size. For example, in another embodiment, each winding may have two interconnections and six peaks or crowns, thereby forming a smaller diameter stent. The interval or number of interconnections may depend on the target stent diameter or the desired cell size.

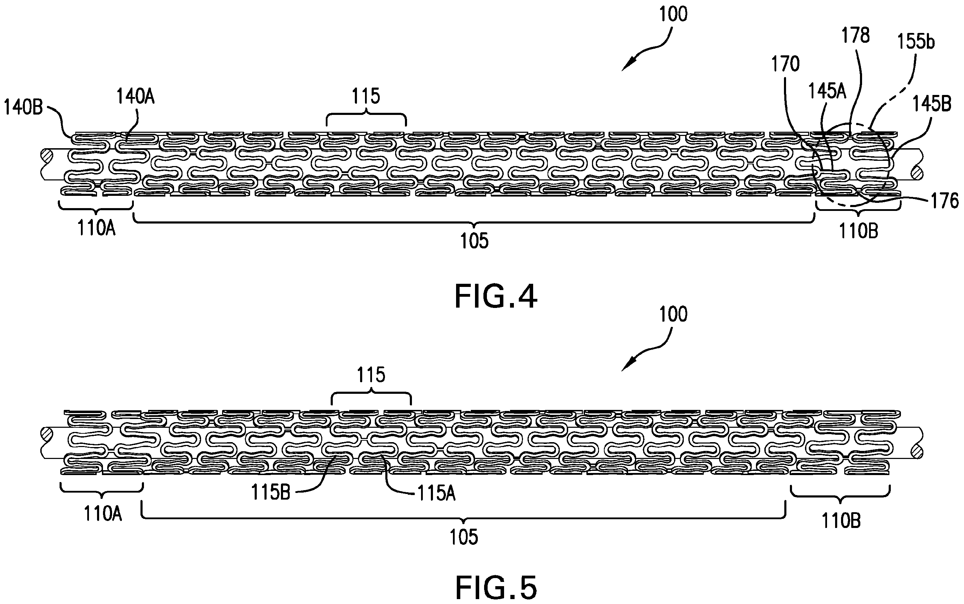

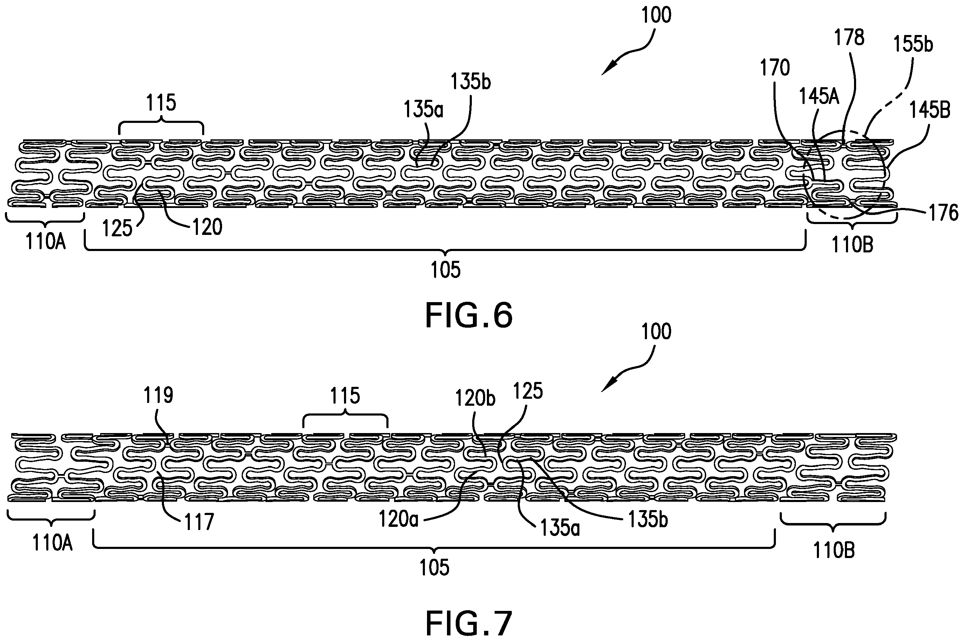

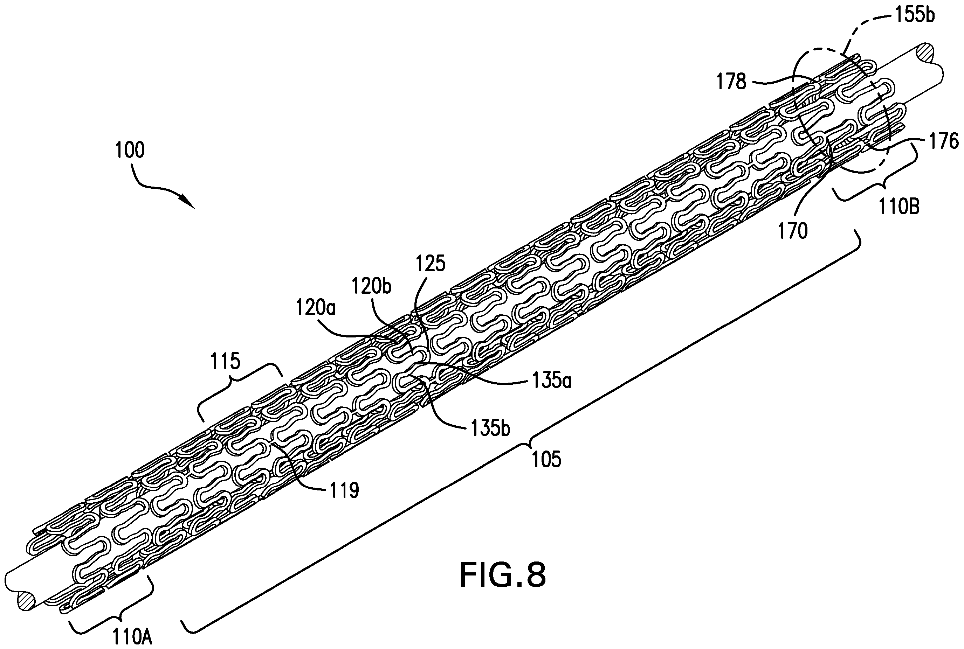

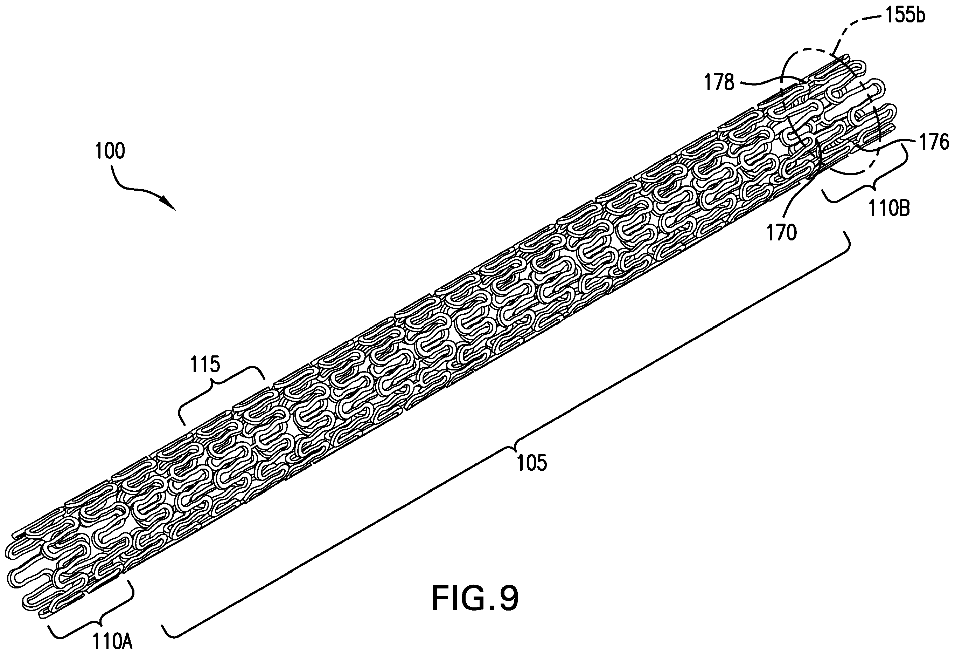

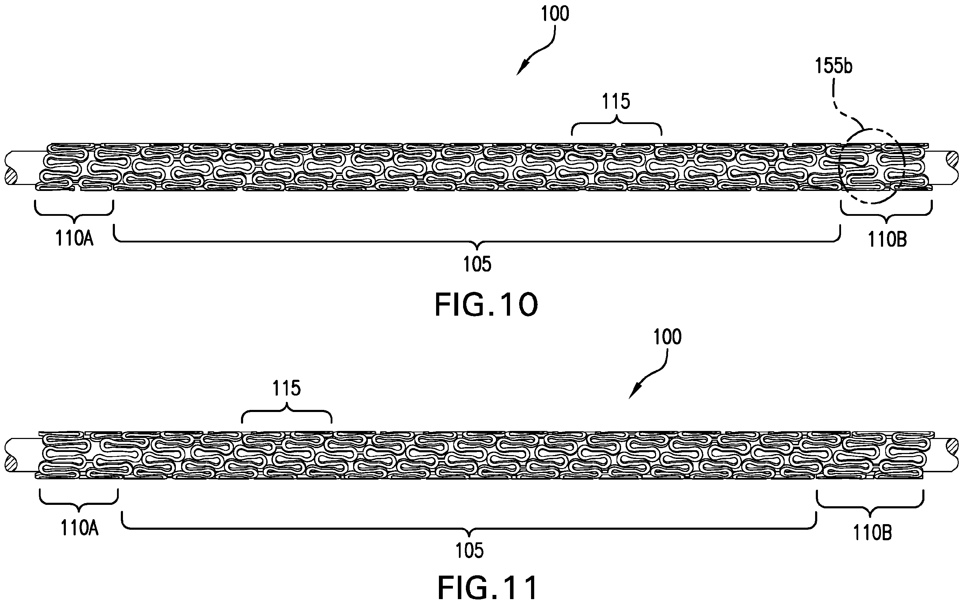

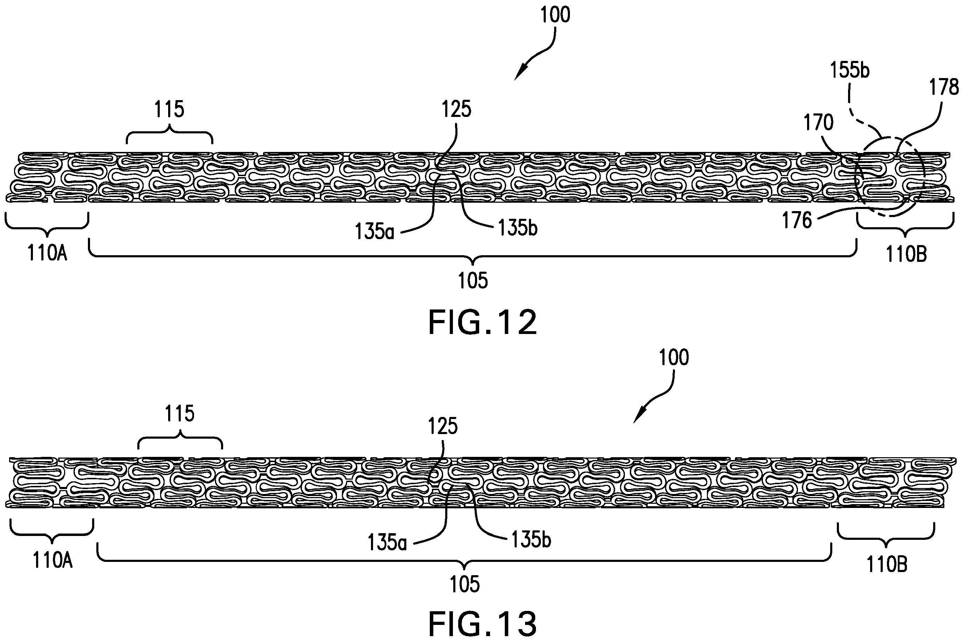

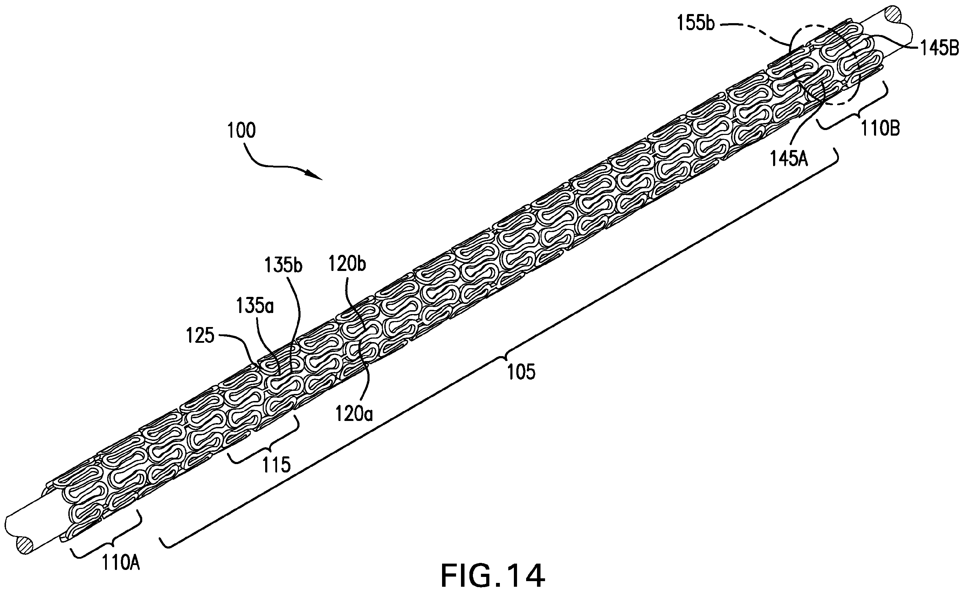

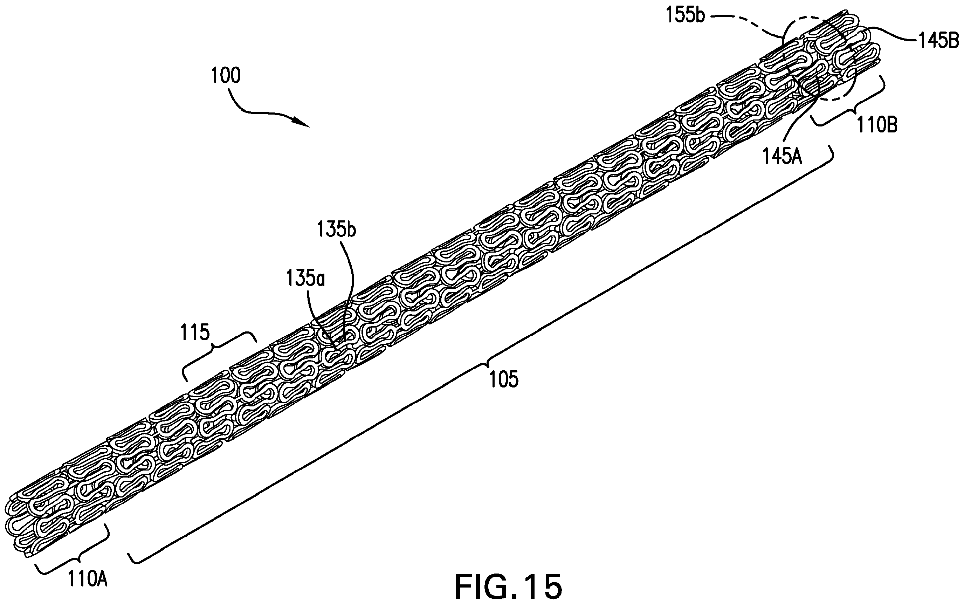

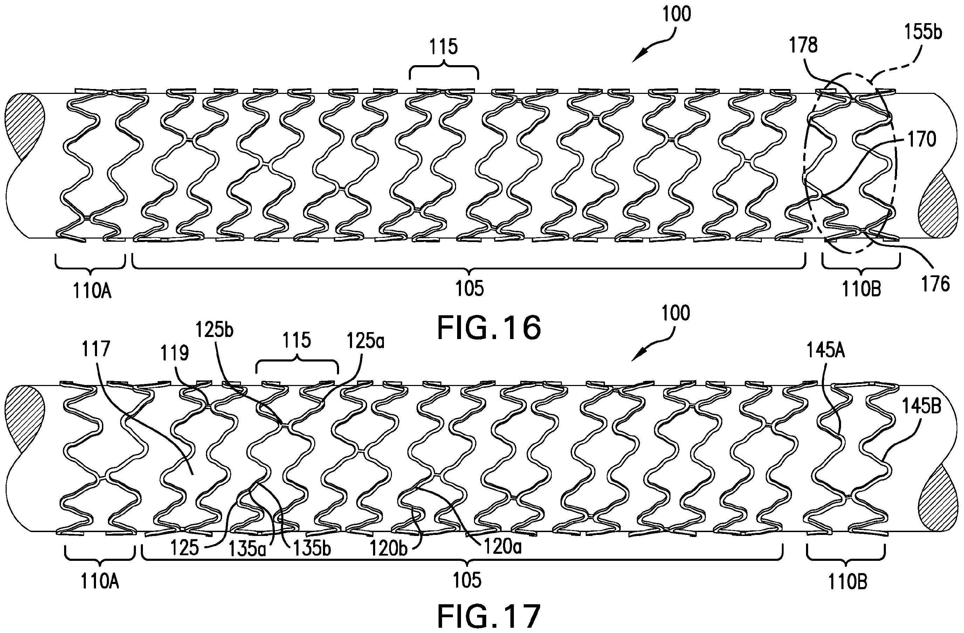

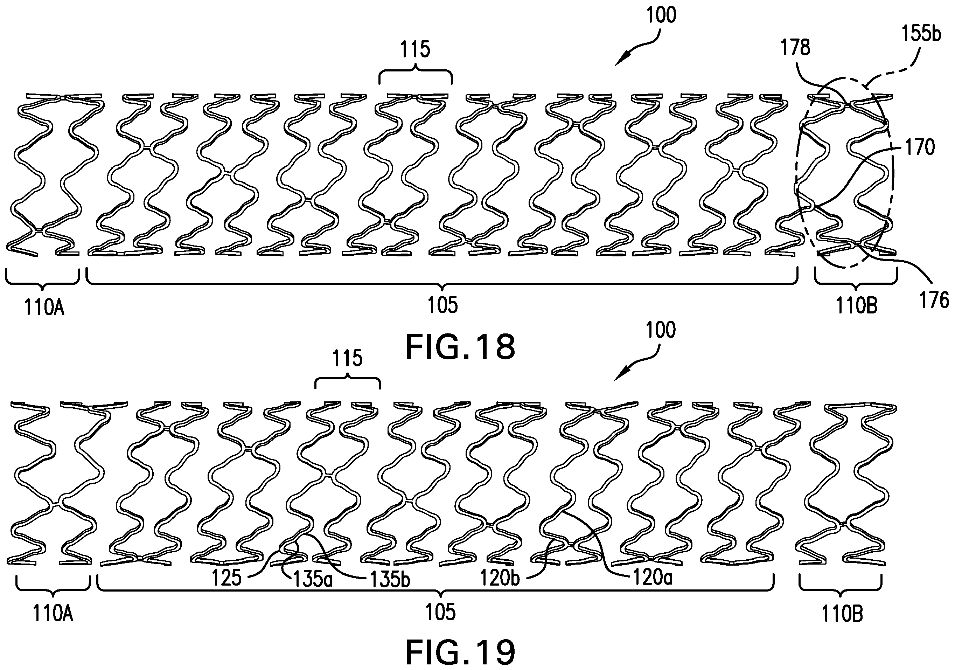

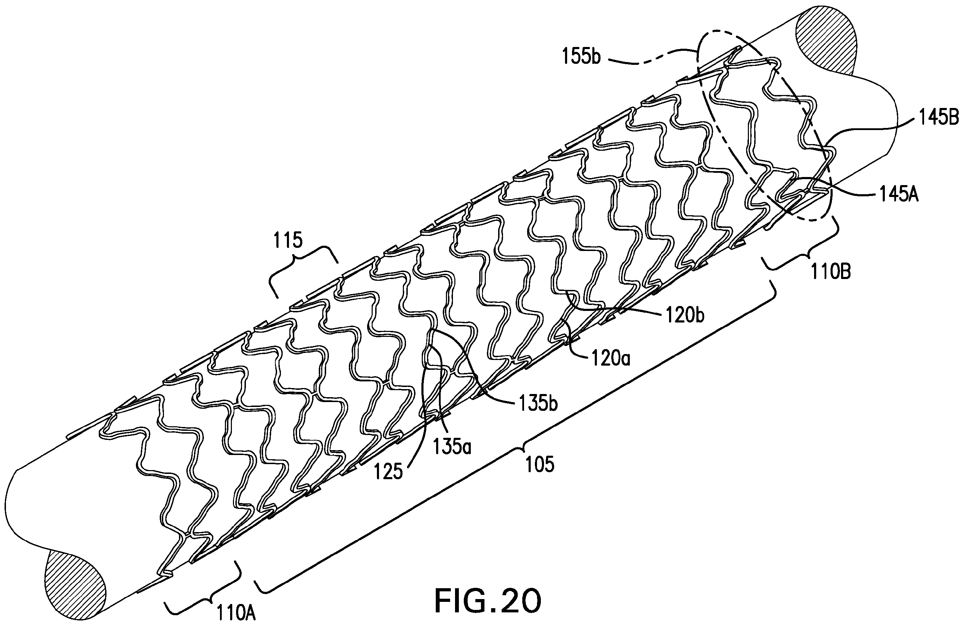

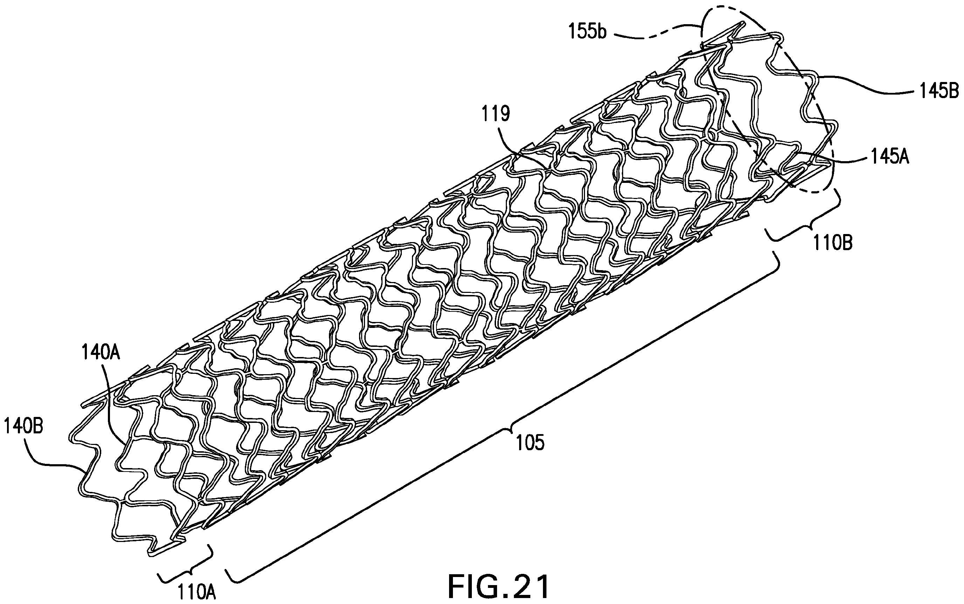

FIGs. 4-21 illustrate a 3-dimensional of the stent 100 according to the embodiment shown in FIG. 3. FIGs. 4-9 illustrate the as-cut configuration of the stent 100 from different perspectives, FIGs. 10-15 illustrate the crimped, delivery configuration of the stent 100 from different perspectives, and FIGs. 16-21 illustrates the expanded, deployed configuration of the stent 100 from different perspectives.

The features of the present invention described herein, individually or in combination, advantageously achieve an enlarged expanded outside diameter and/or a reduced compressed outside diameter compared to conventional stents. For example, in embodiments of the present invention having a combination of long and short struts, an enlarged expanded diameter may be achieved by the long struts. A reduced compressed diameter may be achieved, for example, by the bent strut design contributing to the nestled arrangement in the crimped configuration of the stent. Alternatively or in addition, other features which may further contribute to a reduced compressed diameter include, for example, the staggered or offset pattern of loops which may result from helically orientated windings and/or variable length struts, for example. Further, compared to conventional stents, the stent of the present invention advantageously maximizes the distance between adjacent struts, thereby minimizing the interaction there-between, where such interaction may be harmful to the stent, the stent coating and/or the inner balloon.

The embodiments shown in FIGs. 4-21 are substantially similar to the embodiment shown in FIGs. 1-2, except all of the struts 120 of the main stent component 105 and of the first and second end rings 110A-B have the bent strut design of the first and second bent sections 135a-b. It should be noted, that in any of the embodiments of the present invention, the stent 100 may include the bent strut design in all, some or one strut depending, for example, on the particular application and/or desired expanded or compressed stent diameter size.

Further, similar to FIG. 1, the embodiments shown in FIGs. 4-21 include the windings 115 comprising the two interconnected bands 115A-B, and the two interconnected circumferential end bands 140A-B, 145A-B at the lengthwise ends 100a-b of the stent 100. The embodiments shown in FIGs. 4-21 also illustrate the staggered or offset pattern of adjacent loops 125 in the helical direction H, as similarly described with respect to FIG. 1, as well as struts 120a-b of variable length. Also similar to FIG. 1, FIGs. 4-21 show the cellular design of cells 117 and links 119, however as described above, the some or all of the links 119 may be replaced by direct connections or other connection means. The stent 100 of FIGs. 4-21 may also include the optimal redistribution of the stress/stain concentrations as discussed above, where, for example, all, some or one of the loops 125 may have a width wider than the struts 120 and/or all, some or one of the struts 120 may have a variable width along the strut length. Additionally, the embodiment of FIGs. 4-21 may include one or more transition cells, as discussed above. For example, the third transition cell 155b is shown in FIGs. 4, 6, 8-10, 12, 14-16, 18, and 20-21. The stent 100 of FIGs. 4-21, as well as of any of the other embodiments described herein, may have one, some or all of the features described herein.

FIGs. 4-9 illustrate the stent 100 in the as-cut configuration. The as-cut configuration of the stent 100 is the manufactured profile of the stent 100 when laser cut from a tube or when laser cut or chemically etched from a flat metal sheet which is then rolled into and secured as the tubular form. The outside diameter of the stent 100 in the exemplary as-cut configuration of FIGs. 4-9 is smaller than in the expanded, deployed configuration of FIGs. 16-21 and larger than in the crimped, delivery configuration of FIGs. 10-15, however, it is understood that the stent of the invention may be cut to any desired outside diameter for the as-cut configuration.

As shown in FIGs. 4-9, at least one strut 120 of the stent 100 includes the bent strut design of the first and second bent sections 135a-b. Further, as shown in FIGs. 4-9, loops 125 in the helical direction H are arranged in a non-overlapping (*e.g.*, staggered) relationship. That is, loops 125 are aligned with struts 120 in the helical direction H. In particular, a bent section 135a-b of a strut 120 is aligned with an adjacent loop 125 in the helical direction H. In the as-cut configuration, the loops 125 are aligned but distanced from the bent sections 135a-b, such that the loops 125 are not yet nestled in the opposing bent sections 135a-b.

FIGs. 10-15 illustrate the stent 100 in the crimped, delivery configuration (or partially crimped configuration). The outside diameter of the stent 100 in the crimped (or partially crimped) configuration is smaller than in the expanded, deployed configuration. In the crimped configuration, the struts 120 move closer to each other when the stent 100 is compressed onto a catheter, such as a balloon or an expandable member of a catheter. Alternatively or in addition, a radius of curvature of the loops 125 may decrease as the stent 100 is compressed to the crimped configuration.

As shown in FIGs. 10-15, at least one strut 120 of the stent 100 includes the bent strut design of the first and second bent sections 135a-b. Because loops 125 are aligned with the bent sections 135a-b of the struts 120, the crimping process moves a loop 125 toward the complementary shaped opposing bent section 135a-b of an adjacent strut 120 in the helical direction H, thereby achieving the nestled arrangement. The stent 100 is able to more tightly crimp than a conventional stent because of the achieved nestled arrangement in the crimped profile, where the bent sections 135a-b do not substantially straighten during or when compressed. In particular, stent 100 is able to more tightly crimp than conventional stents because the bent section 135a-b creates a space in which the adjacent loop 125 may nestle. The space created results in a bent shape 130 where an inner distance between a pair of struts 122 is reduced at the inward curvature of the strut 120. As shown in FIGs. 10-15, a loop 125 is tightly crimped or compressed into contact with, or near contact with, a first or second bent section 135a-b. The loop 125 is offset from an adjacent loop (to form offset loops in the helical direction H), such that the loop 125 is positioned proximal with respect to a loop above and distal with respect to a loop below, or vice versa, and therefore interference between adjacent loops 125 are avoided.

In one embodiment, first and/or second bent sections 135a-b maintain the same amount of curvature as in the as-cut configuration (or expanded configuration), such that as the struts 120 move closer to each other during or when compressed (onto a guide catheter) the curvature of the first and/or second bent sections 135a-b do not change. In another embodiment, during or when crimped, the first and/or bent sections 135a-b become more bent, such that curvature of the first and/or second bent sections 135a-b increases, and thereby further reduces the compressed diameter of the stent 100. In yet another embodiment, during or when crimped, the first and/or second bent sections 135a-b become less bent but maintain at least some amount of curvature and do not substantially straighten in order to allow for the nestled arrangement of struts and loops.

FIGs. 16-21 illustrate the stent 100 in the expanded, deployed configuration (or partially expanded configuration). The outside diameter of the stent 100 in the expanded (or partially expanded) configuration is larger than in the crimped, delivery configuration. In the embodiments shown in FIGs. 16-21, the stent 100 is expanded or deployed to a 3.0 mm outside diameter, however other diameters may be possible as suitable for a particular application. In the deployed configuration, as the stent 100 is expanded by a guide catheter, such as a balloon or expandable member of a catheter, or is self-expanded, the struts 120 move away from each other so that a radius of curvature of the loops 125 is increased.

As shown in FIGs. 16-21, at least one strut 120 of the stent 100 includes the bent strut design of the first and second bent sections 135a-b. Further, as shown in FIGs. 16-21, the loops 125 in the helical direction H remain in the non-overlapping (*e.g*., staggered) relationship, where loops 125 are aligned with (but distanced from) the bent section 135a-b of the struts 120.

In one embodiment, first and/or second bent sections 135a-b maintain the same amount of curvature as in the as-cut configuration or crimped configuration such that, as the struts 120 move away from each other during or when expanded, the curvature of the first and/or second bent sections 135a-b do not change. In another embodiment, during or when expanded, the first and/or bent sections 135a-b become more bent, such that curvature of the first and/or second bent sections 135a-b increases. In yet another embodiment, during or when expanded, the first and/or second bent sections 135a-b become less bent but maintain at least some amount of bend or curvature and do not substantially straighten. In still a further embodiment, during or when expanded, the first and/or second bent sections 135a-b straighten or substantially straighten, thereby further enlarging the expanded diameter of the stent 100.

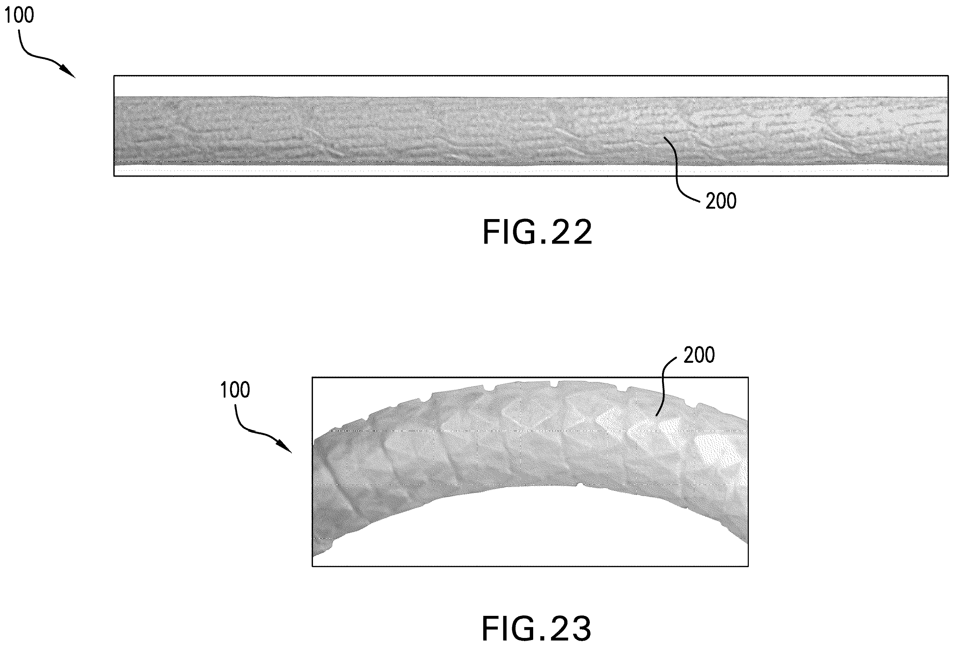

FIGS. 22-23 depict the stent 100, according to any of the embodiments discussed herein, with an optional polymer coating 200. The polymer coating 200 may be made from or include a biodegradable or biocompatible polymer and/or may include a drug, for example, in a formulation. Moreover, the polymer coating 200 may be in the form of a fiber mesh. The polymer coating 200 may be applied by, for example, electrospinning, physical vapor deposition (PVD), chemical vapor deposition (CVD), thermal evaporation, sputtering, spray coating, dip-coating or other methods known in the art. The polymer coating 200 may be applied to all or a portion of the stent 100 in a continuous or non-continuous manner, and may or may not embed the stent 100. In one embodiment, the polymer coating 200 may be applied or extends in a gap between adjacent windings 115 and/or in a gap formed by a cell 117. The elastic range of the polymer coating 200 (*e.g*., mesh of fibers) is preferably sufficient to allow expansion of the stent 100 and maximal bending during and after implantation without reaching the elastic limit. Further, the polymer coating 200 may be substantially porous such that blood flow and nutrient flow is permitted therethrough or the polymer coating 200 may be applied to the stent 100 in a manner permitting the stent 100 to be a substantially porous structure (*i.e*., not fluid-tight). The porosity value of the polymer coating 200 is substantially greater than that of a graft material used in graft or stent-graft devices which are substantially fluid-tight. Alternatively, the material of the polymer coating 200 may be entirely non-porous, but may be made (*e.g*., punctured) to include openings for blood and nutrient flow and/or side branch access, for example.

In one embodiment, the polymer coating 200 may be formed as a continuous sheet of polymer material over the stent 100. The continuous sheet may be a porous sheet enveloping an outer surface of the stent or embedding the stent therein. The polymer coating 200 may be made porous through pores and/or fenestrations formed on the continuous sheet. The pores and/or fenestrations may or may not be irregularly shaped, and may or may not be uniformly distributed throughout the stent 100. The pores and/or fenestrations may be formed on portions of the continuous sheet which do not envelop or embed the structural components (*e.g*., struts 120 and loops 125) of the stent 100. The size of the pores may be in a range of 2.0 to 500 microns, and the size of the fenestrations may be larger than the pores. In another embodiment, the polymer coating 200 may be a mesh of fibers having a porous structure permitting fluid flow therethrough. The mesh of fibers may themselves be porous or may be arranged at variable distances (*e.g*., interstices) to provide for the non-fluid tight stent 100 having sides that allow fluid flow (i.e, fluid flow through the cylindrical envelope of the stent). The polymer may interconnect adjacent helical windings of the stent, whether or not such adjacent windings of the backbone of the stent are unconnected or connected in the longitudinal direction by flexible connectors. In any of the above embodiments, the polymer may interconnect one or a plurality of windings of the stent. In some embodiments, the polymer interconnects every winding of the stent. The polymer coating 200 may be easily pierced, by for example a catheter or guidewire tip, to allow for side branch access. The polymer coating 200 may be applied in-between structural portions of the stent 100 and/or may be coated onto the structural portions of the stent 100 such as coated onto the stent struts. One skilled in the art recognizes methods of coating, *e.g*., as described in U.S. Patent No. 7,959,664 entitled "Flat Process of Drug Coating for Stents" the entire contents of which are incorporated herein by reference.

The stent of the invention may be formed from metals, polymers, other flexible materials and/or other biocompatible materials. The stent may be constructed of stainless steel, cobalt chromium ("CoCr"), platinum chromium, NiTinol ("NiTi") or other known materials or alloys. The stent pattern or design as described herein may be etched or laser cut into a flat metal ribbon or a flat panel. Alternately, the stent may be made from a tube wherein the stent pattern or design has been etched or laser cut into it. In either case, the stent will have a pattern resembling the embodiments described herein. It is also contemplated that the stent may be formed from helically winding a flat strip or wire having the stent pattern or design described herein. In one embodiment, the invention contemplates wrapping or embedding the stent with a biocompatible polymer such that the polymer may structurally support the stent but does not limit longitudinal and/or twisting movement of the stent, thereby forming a stent with high radial strength and high longitudinal (*i.e*., lengthwise) flexibility.

In one embodiment, the stent of the invention is a hybrid stent where the radial (tubular/helical/spiral) structure is provided by a metallic backbone of the main stent component, and the longitudinal structure is provided by a polymer mesh. The metallic backbone may be made of CoCr. The polymer mesh may be a biodegradable polymer mesh of fibers comprising DL-lactide/glycolide copolymer (PDLG) and poly-DL-lactide (PLC). The polymer mesh provides a mechanical role, namely, to structurally supports the longitudinal structure of the stent. In addition, the mesh may provide a functional role, such as to provide a controlled drug elution bed, thereby resulting in drug eluting stent (DES). The biodegradable polymer mesh may be electrospun over the metallic backbone of the stent.

The stent of the invention may be balloon expandable or self-expanding. When a balloon-expandable stent system is used to deliver the stent, the stent is crimped on a balloon at the distal end of a catheter assembly which is then delivered to the implantation site, for example a coronary artery, using techniques well-known in the field of interventional cardiology. The balloon is then inflated, radially applying a force inside the stent and the stent is expanded to its working diameter. Alternatively, the stent may be self-expanding in which case the stent is held in a restricted diameter before and during delivery to the implantation site using mechanical means, for example a sleeve. When the stent is positioned in the implantation site, the sleeve is removed and the stent expands to its working diameter.

The stent may be arranged to provide a cellular stent design, where the cells are formed between the first and second bands, between a helical band and a circumferential end band, and/or between first and second circumferential end bands. Example designs are described in, but not limited to, U.S. Pat. No. 6,723,119, which is incorporated herein *in toto,* by reference. Another example design is a stent pattern described in U.S. Pat. No. 7,141,062 (‴062"). The '062 stent comprises triangular cells, by which is meant a cell formed of three sections, each having a loop portion, and three associated points of their joining forming each cell. One or more rows of such cells may be assembled in a ribbon which may be helically coiled from the stent. Similarly, the cells in the stent described in U.S. Pat. No. 5,733,303 to Israel et al. (‴303") may be used for the stent but helically coiled. The '303 patent describes a stent having cells formed of four sections, each having a loop portion and four associated points of their joining forming each cell, also known as square cells. Such square cells may be formed with the first and second bands and links of the stent of the present invention. Each of these designs is expressly incorporated herein *in toto* by reference. Other similarly adaptable cellular stent designs known in the art are readily applicable to the helical stent of the present invention, such as diamond shaped cells or non-diamond shaped cells.

A basecoat may optionally be applied to the stent of the present invention. The basecoat is applied on the structural structure of the stent and prior to applying the optional polymer coating or material. The basecoat may promote the joining of the optional polymer material to the stent. The basecoat may be applied or affixed to the stent through a variety of means, for example, rolling, dip coating, spray coating or the like. The basecoat may be a polymer, such as a bio-stable or a biodegradable polymer. The bio-stable polymer used for the basecoat may be a polyurethane or an acrylate type polymer. The biodegradable polymer used for the basecoat may be the same or different than the biodegradable polymer used to wrap or embed the stent, such as the polymer coating 200 of FIGs. 22-23. The polymer for the basecoat is selected to be adhesive to the structural structure of the stent at specific conditions, while the polymer for wrapping or embedding the stent may adhere to the basecoat at different conditions. The polymer for the basecoat may be dissolvable in most solvents and should be flexible enough to withstand significant deformations during and after stent deployment.

The polymer used to optionally wrap or embed the stent, such as the polymer of FIGs 22-23, can be disposed within parts of the stent or embedded throughout the stent and it may support the stent structure partially or fully. The polymer is made from a biocompatible material. Biocompatible material may be a durable polymer, such as polyesters, polyanhydrides, polyethylenes, polyorthoesters, polyphosphazenes, polyurethane, polycarbonate urethane, silicones, polyolefins, polyamides, polycaprolactams, polyimides, polyvinyl alcohols, acrylic polymers and copolymers, polyethers, celluiosics and any of their combinations or combination of other polymers in blends or as copolymers. Of particular use may be silicone backbonemodified polycarbonate urethane and/or expanded polytetrafluoroethylene (ePTFE). Alternatively, the biocompatible material may be a biodegradable polymer. The polymer may be a porous mesh of polymer fibers. The polymer may further include an antiproliferative drug which inhibits growth of smooth muscle cells and helps prevent restenosis (re-narrowing of the vessel) at the stent implantation site. The combination of drug and polymer offers the advantage of controlled drug elution over a predetermined period of time (*e.g.*, 30, 60, or 90 days) depending on the requirements of a particular procedure and the technical characteristics of the polymer. The drug elution may be controlled by, for example, the selection of the polymer or blend of polymer(s) and drug, the polymer mesh dimensions, fiber diameter or fiber structure, or any structural feature which affects the coefficient of diffusion. The biodegradable polymer may be selected so as to completely biodegrade within the vessel wall over a predetermine period of time and after drug elution has completed. The biodegradable polymer may be selected from the group consisting of: polyglycolide, polylactide, polycaprolactone, polydioxanone, poly(lactide-co-glycolide), polyhydroxybutyrate, polyhydroxyvalerate, trimethylene carbonate, polyphosphoesters, polyphosphoesterurethane, polyaminoacids, polycyanoacrylates, fibrin, fibrinogen, cellulose, starch, collagen, hyaluronic acid and blends, mixtures and copolymers thereof.

In one embodiment, the biodegradable polymer is a mesh of fibers comprising DL-lactide/glycolide copolymer (PDLG) and/or poly-DL-lactide (PLC). The use of PDLG and PLC is advantageous over other biodegradable polymers because they provide advantageous characteristics including biocompatibility, control of drug elution, rate of degradation of the polymer itself and mechanical properties. The polymer mesh provides a mechanical role, namely, to structurally support the longitudinal structure of the stent. In addition, the mesh may provide a functional role, such as to provide a controlled drug elution bed as described above. The biodegradable polymer mesh is created by electrospinning, resulting in a mesh structure having a plurality of polymer fibers each having a fiber diameter of approximately 3-5 microns and with a majority of the pores being large pores greater than 100 µm² in between the fibers. The mesh structure advantageously achieves a dual purpose: (i) blood flows through the pores is unimpeded such that, unlike a covered stent, the mesh does not block flow into side branches and (ii) cells can migrate freely through the mesh. In one embodiment, within one to two weeks after implantation, the stent together with the mesh is entirely incorporated into the vessel wall, thereby obviating the mechanical role of the mesh, which begins to degrade and is completely degraded within 3 months.

The stent according to the invention may be a drug eluting stent (DES) that may optionally incorporate one or more drugs that will inhibit or decrease smooth muscle cell migration and proliferation, and reduce restenosis. Examples of such drugs include for example rapamycin, paclitaxel, sirolimus, everolimus, zotarolimus, ridaforolimus, biolimus, and analogs thereof. The drug may be provided on the structural portions of the stent (*e.g*., struts and/or loops) and/or on the polymer material or coating (*e.g*., polymer coating 200 of FIGs. 22-23). For example, the drug may be provided as a partial or complete coating over the stent 100 and/or the polymer coating 200. The stent may be surface-treated to have abnormalities (*e.g*., indentations, wells or fenestrations) which may house the drug therein or thereon. Alternatively or in addition, the polymer material may have abnormalities (*e.g*., indentations, wells or fenestrations) for housing the drug therein or thereon.

In one embodiment, a rapamycin analog drug (e.g., ridaforolimus) is incorporated into the polymer fibers of the biodegradable polymer fiber mesh and is released with a controlled drug elution profile over a 1-3 month period. The drug is provided as a complete coating of the stent as the polymer fiber mesh is electrospun over the entirety of the backbone of the stent, as described above. Drug elution or release from the entire stent surface and directly from the polymer fibers themselves, rather than from the stent backbone, advantageously assures drug release with improved drug uniformity within the vessel over the entire stent surface and further advantageously reduces or essentially eliminates drug diffusion distances within the vessel wall. This may thereby allow for a reduction in drug dose, such as a reduction of greater than 4 fold, relative to conventional drug eluting stents (DES), while still maintaining an efficacious concentration of drug within the vessel wall over an extended period of time. In one embodiment, the polymer fiber mesh includes a drug dose amount of approximately 20-25 micrograms for a 15 mm long stent or about 0.2-0.3 micrograms of drug per mm² stent surface area, for example.

In an embodiment, the drug may be selectively "printed" on targeted regions of the structural backbone portions of the stent and/or polymer material portions. In one embodiment, the drug may be confined to and/or provided on only those regions subjected to lower mechanical strain after implantation. In one embodiment, the drug or drug/polymer formulation is deposited or printed using an inkjet technique. An inkjet device includes an inkjet head with a small orifice. When voltage is applied to the inkjet device, it contracts for a period of milliseconds and spits out a small drop of desired product (*e.g*., drug or drug/polymer formulation). The drop diameter is adjustable and varied, and moving the inkjet head or the target object (*e.g*., stent) provides for the selective and precise product deposition/printing.

It is desirable to design the structure of the stent such that after neointimal growth and "embedding" of the stent struts in the tissue, the stent does not interfere with vasomotion of the blood vessel in which it is implanted. This reduction or elimination in interference is achieved by reducing the mechanical resistance of the stent to bending/twisting (*i.e*., via the stent design and/or the polymer coating), as well as to expansion/contraction. The exemplary stent configurations described herein provide stents which support the blood vessel radially, but impose minimal mechanical constraint longitudinally and torsionally. Specifically, the inventive stent imposes minimal mechanical constraint on the transverse flexion, torsion, elongation and vasodilatation/vasoconstriction (e.g., expansion/contraction) of the blood vessel.

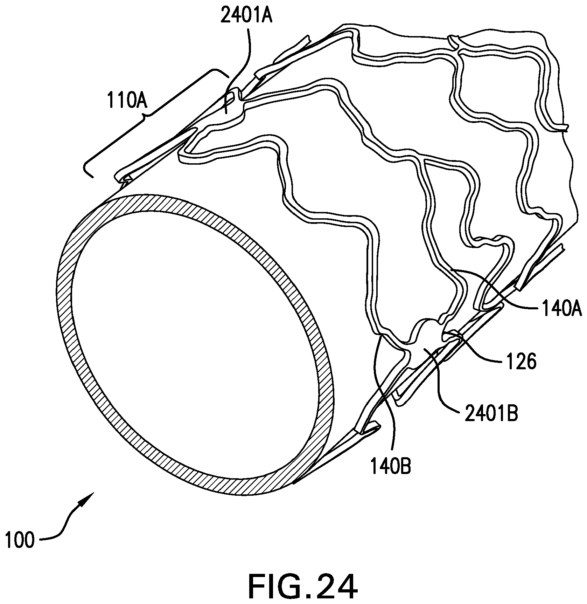

In one exemplary embodiment which illustrates an embodiment using both the first and second aspects of the invention (shown e.g., in FIG. 24), two radiopaque markers are located at a first end of the stent and are offset relative to each other by an angle less than 180 degrees, and two other radiopaque markers are positioned at a second end of the stent are also offset relative to each other by an angle less than 180 degrees. FIG. 24 shows a partial perspective view of a stent having two radiopaque markers 2401A, 2401B at one end of the stent 100 that are offset by 90 degrees relative to each other such that the two radiopaque markers 2401A, 2401B may be advantageously observed head on during angiographic and/or radiographic imaging to enable improved stent navigation and placement within a vessel. The two radiopaque markers 2401A, 2401B, shown in FIG. 24, are circular in shape. As shown in the embodiment of FIG. 24, the two radiopaque markers 2401A, 2401B at the end of the stent 100 are mounted on a loop of an end ring 110A. In this embodiment, the radiopaque markers 2401A, 2401B are positioned at attachment loops 126 between the two interconnected circumferential end bands 140A-B, 145A-B, as shown in FIG. 24. Thus, in addition to providing radiopacity, the radiopaque markers may also provide an indirect connection means connecting a loop of the first circumferential end band 140A, 145A to an adjacent loop of the second circumferential end band 140B, 145B. The plurality of the radiopaque markers mounted on a loop of the second end ring 110B is not shown but is substantially similar to the radiopaque markers 2401A, 2401B mounted on the first end ring 110A shown in FIG 24. While two markers are shown at the first end ring 110A, more than two markers may be mounted on one or both of the end rings, 110A, 110B. Further, the number of markers on the first and second end rings 111A, 110B may the same or different.

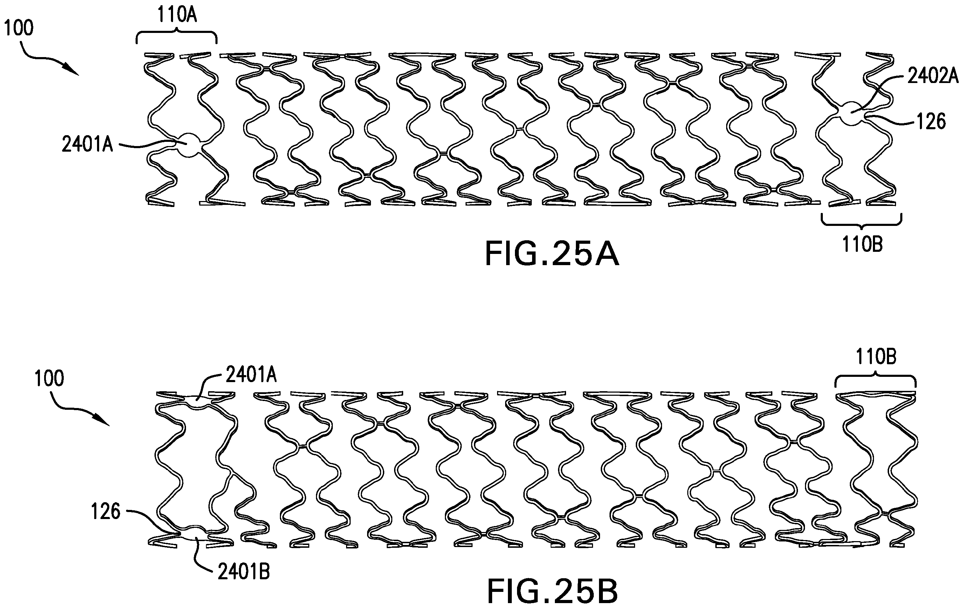

FIG. 25A-B illustrate a planar view of the stent 100 of FIG. 24 along the longitudinal axis from a first end to a second end of the stent 100. As shown in FIG. 25A, the radiopaque marker 2401A on the first end ring 110A is offset relative to radiopaque marker 2402A on the second end ring 110B. Radiopaque marker 2401A of the first end ring 110A may be offset by less than 180 degrees, such as by about 90 degrees, 120 degrees, or 45 degrees, relative to radiopaque marker 2402A of the second end ring 110B. FIG. 25B is a rotated view of FIG. 25A and shows the two radiopaque markers 2401A, 2401B positioned at the attachment loops 126 of the first end ring 110A. It should be noted the end rings 110A, 110B of FIGs. 25A-B each include at least two radiopaque markers, but are not all visible in FIGs. 25A-25B. Further, it should be noted that the radiopaque markers of FIGs. 24-25B may be incorporated in any type of stent and is not limited to a helical or spiral stent design, and is further not limited to be used in combination with the other features described in the above embodiments of FIGs. 1-23.

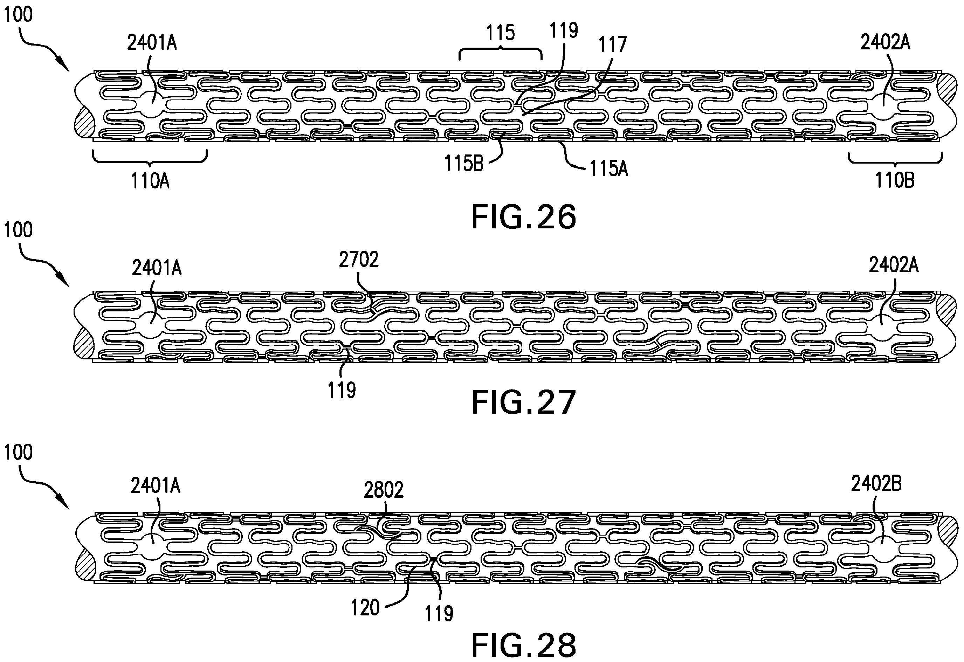

FIGs. 26-28 illustrates a stent, in a planar view, according to the other embodiments of present invention. The stent of FIGs. 26-28 is in the as-cut configuration. The stent of the embodiments shown in FIGs. 26-28 may include all or some of the features in any combination, as described above and/or in relation to the embodiments shown in FIGs. 1-25B, including but not limited to all or some of the features of the main stent component 105, the end rings 110A-B, the polymer coating 200, the controlled drug elution, the bent strut design, the staggered pattern of alignment of adjacent loops, and the plurality of radiopaque markers.

The embodiment shown in FIG. 26 is substantially similar to the embodiments shown in FIGs. 1-2 and FIGs. 4-21, except FIG. 26 further illustrates the plurality of radiopaque markers 2401A, 2402A located on the end rings 110A-B as described above and shown in FIGs. 24 and 25A-B. It should be noted that not all of the radiopaque markers are visible in FIG. 26. Further, similar to FIGs. 1-2, FIGs. 4-21 and FIGs. 24, 25A and 25B, the stent 100 shown in FIG. 26 illustrates an embodiment where adjacent windings of the stent 100 are unconnected in the longitudinal direction of the stent by a link or direct connection such that no enclosed cells are formed between adjacent windings along the coiled pattern of the stent from the first end to the second end of the stent. Further, as shown in FIG. 26, and as similarly described above in relation to FIGs. 1-2 and FIGs. 4-21, the stent 100 comprises two interconnected bands 115A-B within an individual winding 115 thereby forming enclosed cells 117 within each individual winding 115. The first and second bands 115A, 115B are shown in FIG. 26 to be interconnected by an indirect connection, such as by links 119, however, other interconnection means may be provided as described above such as by a direct connection. This structure of a plurality of interconnected bands 115A-B within an individual winding and adjacent windings being unconnected (i.e., no indirect links or direct connections in the longitudinal direction) allows for exceptional improved flexibility, preventing vessel straightening and allowing vessel flexion within, for example, the cardiac cycle. It should be noted that in another embodiment (not shown), the stent may comprise a single band within each winding (instead of a plurality of interconnected bands within a winding) and where adjacent windings are unconnected in the longitudinal direction such that no enclosed cells are formed within a winding and further such that no enclosed cells are formed between adjacent windings of the coiled or spiral pattern of the stent.

The embodiment shown in FIGs. 27-28 are substantially similar to the embodiment shown in FIG. 26, except FIGs. 27-28 illustrate an embodiment where adjacent windings of the stent 100 are interconnected by at least one indirect and/or direct connector between one or more adjacent windings. Accordingly, in contrast to the embodiment shown in FIG. 26, the stent 100 shown in FIGs. 27-28 may include enclosed cells 117 within each winding (i.e., enclosed between the interconnected bands 115A-B within an individual winding 115) and further may include enclosed cells between adjacent windings when at least two connectors are present between adjacent windings. It should be noted that when only a single connector is present between adjacent windings then no enclosed cells will be formed between the adjacent windings. Further, it should be noted that the number of connectors between adjacent windings will depend on the particular application of the stent. It should also be noted that the connectors between adjacent windings may be present between all adjacent windings or some of the adjacent windings, such as every other or every third adjacent windings. In addition, the number of connectors between adjacent windings may be the same or may be different than the number of connections (e.g., links 119) within each winding 115 (i.e., between the interconnected bands 115A-B). In one embodiment, the number of connectors between adjacent windings is less than the number of interconnections (e.g., links 119) within each winding 115. Further, it is noted that the connectors between adjacent windings may be similar in structure and design to the interconnections (e.g., links 119 or direct connections) connecting the first and second bands 115A, 115B within each winding as described above with respect to the embodiments shown in FIGs. 1-2 and FIGs. 2-21. FIGs. 27-28 also illustrate the plurality of radiopaque markers 2401A, 2402A located on the end rings 110A-B as described above and shown in FIGs. 24 and 25A-B. It should be noted that not all of the radiopaque markers are visible in FIGs. 27-28, and should further be noted that the stent 100 of FIGs. 27-28 need not include any radiopaque markers.

In one exemplary embodiment, as shown in FIG. 27, the stent 100 includes one or more direct connections between some or all of the adjacent windings. A direct connection may directly connect adjacent bands of adjacent windings at loops of the undulating pattern, which may be referred to as attachment loops of adjacent windings. Such a direct connection between loops of adjacent windings may be referred to as an H-shaped connection 2702. The direct connection may be achieved by any type of direct connection means, such as, fusing, welding, adhesive bonding, soldering, laser welding, mechanical or physical joining, among others. FIG. 27 also illustrates the radiopaque markers 2401A and 2402A as described above with respect to FIGs. 24-25B, and illustrates the features shown in FIGs. 1-2 and FIGs. 4-21 such as the interconnections (e.g., links 119) connecting the first and second bands 115A-115B within an individual winding. However, it should be noted that the H-shaped connectors 2702 between adjacent windings may be incorporated in any stent and is not limited to a helical or spiral stent design, and is further not limited to be used in combination with the other features described in the above embodiments of FIGs. 1-25B.

In another exemplary embodiment, as shown in FIG. 28, the stent 100 includes one or more indirect connections between some or all of the adjacent windings. An indirect connection may be a flexible connector such as a link or cross-strut extending between adjacent bands of adjacent windings. For example, an indirect connection may extend between a first band of a first winding and a second band of a second winding, where the second winding is adjacent the first winding and the first band is adjacent the second band. In the embodiment shown in FIG. 28, the indirect connection connects adjacent struts of adjacent windings, and may be referred to as an S-shaped connection 2802. The struts (of the undulating pattern) at which the adjacent bands of adjacent windings are connected by the S-shaped connector 2802 may be referred to as attachment struts. The S-shaped connector 2802 extends from approximately a center of the attachment strut of the first winding to approximately a center of the attachment strut of the adjacent, second winding, as shown in FIG. 28. However, it should be noted that the indirect connector may be positioned at a different portion of the attachment struts or may be positioned to connect loops of adjacent windings (i.e., to connect attachment loops). Alternatively, the indirect connector may be positioned to connect an attachment strut of a first winding to an attachment loop of an adjacent, second winding. Further, as shown in FIG. 28, the S-shaped connector 2802 is not a linear connector but is a curved connector. The curvature of the S-shaped connector 2802 includes a convex portion and a concave portion such that a loop of the first winding may nestle in the convex portion and a loop of the adjacent, second winding may nestle in the concave portion, for example. Such nestling may occur during or in the crimped configuration of the stent 100. The nestling provided by such an S-shaped connector 2802 contributes to the advantageous low crimping profile of the stent 100. However, it should be noted that other indirect connectors between adjacent windings are within the scope of the invention, such as linear or straight connectors. Further, the indirect connectors between adjacent windings may have the same or different length, thickness and/or width as the struts 120 of the undulating pattern or as the links 119 interconnecting the bands 115A-B within an individual winding 115 as described in the above embodiments of stent 100. In the embodiment shown in FIG. 28, the S-shaped connectors 2802 have a length longer than the length of the struts 120 and longer than the length of the links 119. In one embodiment, the width and thickness of the S-shaped connectors 2802 may be same or smaller than that of the struts 120 and may be the same or smaller than that of the links 119. It should be noted that the S-shaped connectors 2802 between adjacent windings may be incorporated in any stent and is not limited to a helical or spiral stent design, and is further not limited to be used in combination with the other features described in the above embodiments of FIGs. 1-25B such as with the radiopaque markers, with the bent strut design, and/or with the links 119 within an individual winding, among others.

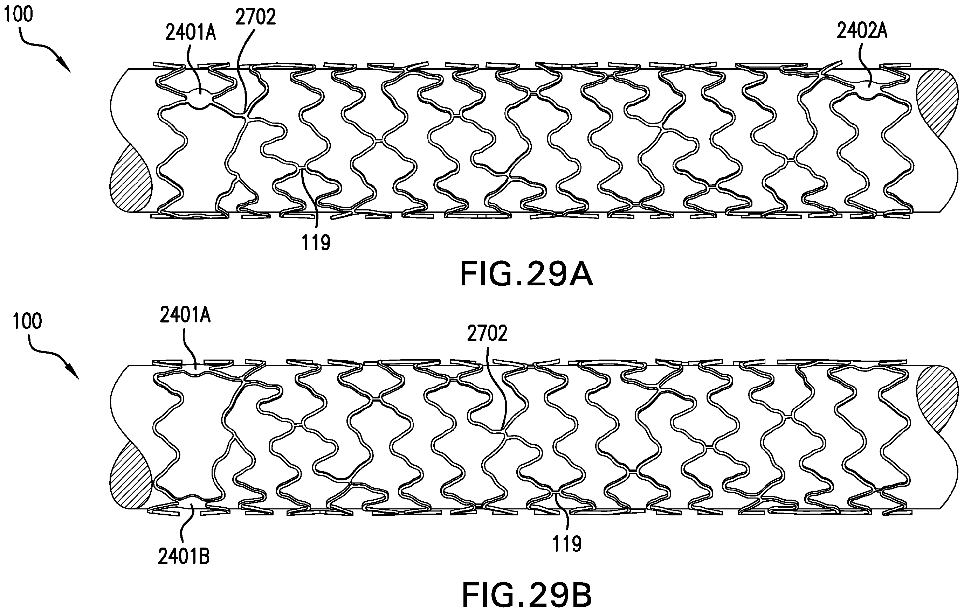

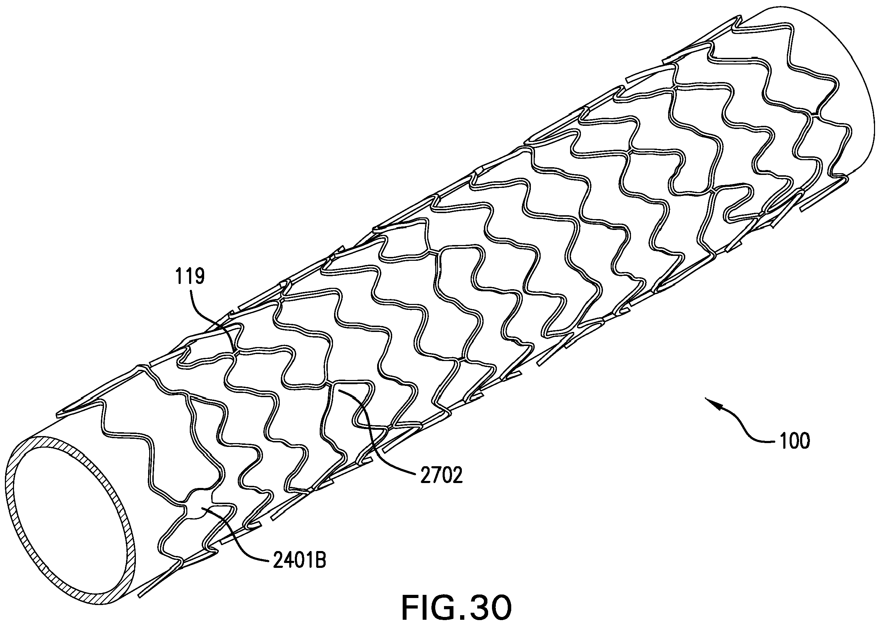

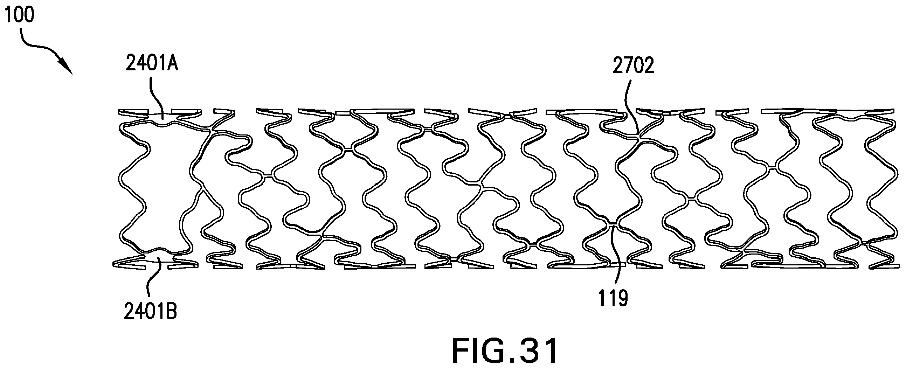

FIGs. 29A-B illustrate the expanded, deployed configuration of a planar view of the stent 100 shown in FIG. 27. FIG. 29A is identical to FIG. 27, except FIG. 29A is in the expanded, deployed configuration and FIG. 27 is in the as-cut configuration. Radiopaque marker 2401A is visible on the first end ring 110A and radiopaque marker 2402A is visible on the second end ring 110B. FIG. 29B is identical to FIG. 29A, except FIG. 29B is rotated about the longitudinal axis such that two offset radiopaque markers 2401A, 2401B are visible on the first end ring 110A. The direct or H-shaped connectors 2702 between adjacent windings are shown in FIGs. 29A-B. Links 119 connecting the interconnected bands 115A-B of an individual winding are also shown in FIGs. 29A-B. FIG. 30 illustrates a perspective or 3-dimensional (3D) view of the stent 100 shown in FIGs. 29A-B. FIG. 31 is identical to the stent 100 shown in FIG. 29B, except FIG. 31 is shown without the inner tube, which may represent an inner mandrel used during stent manufacture, or an inner guide catheter tubing or inner guidewire used during stent delivery, for example.

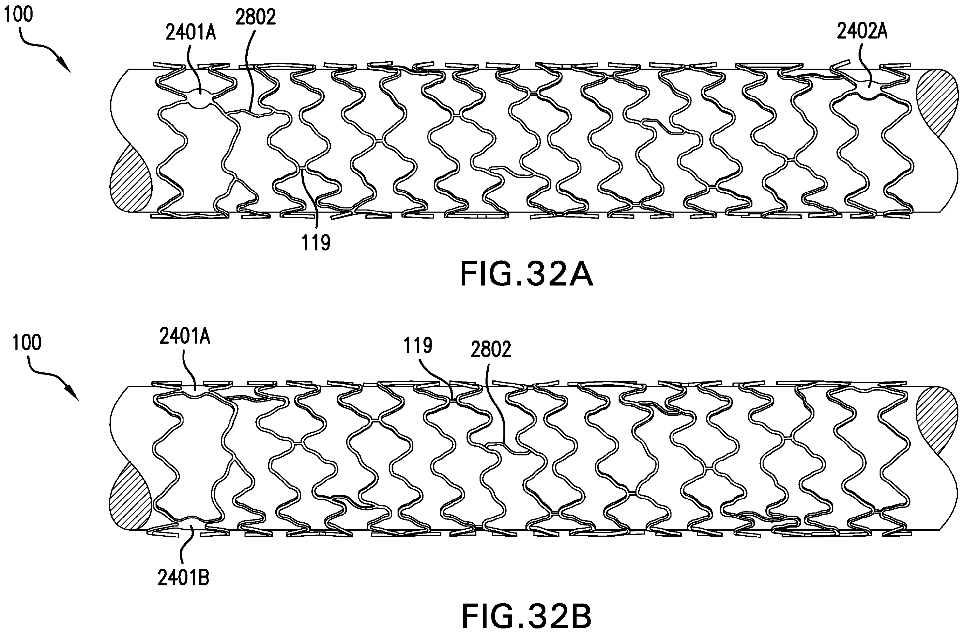

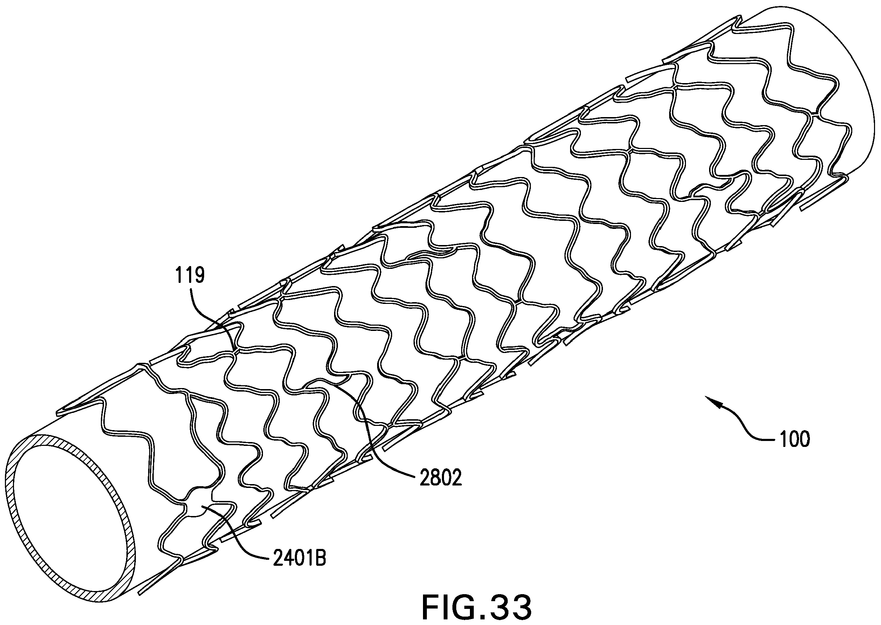

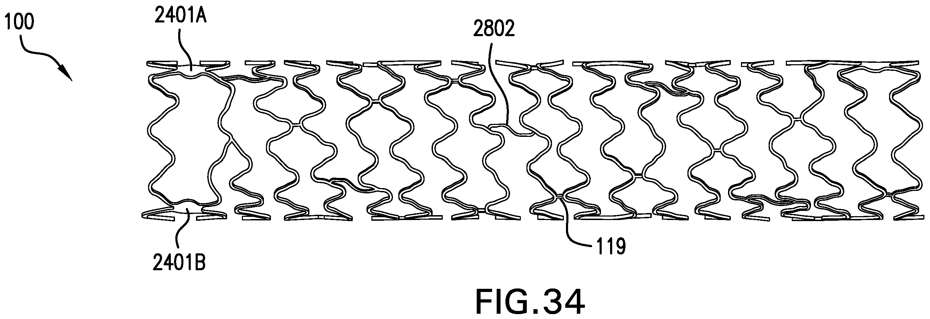

FIGs. 32A-B illustrate the expanded, deployed configuration of a planar view of the stent 100 shown in FIG. 28. FIG. 32A is identical to FIG. 28, except FIG. 32A is in the expanded, deployed configuration and FIG. 28 is in the as-cut configuration. Radiopaque marker 2401A is visible on the first end ring 110A and radiopaque marker 2402A is visible on the second end ring 110B. FIG. 32B is identical to FIG. 32A, except FIG. 32B is rotated about the longitudinal axis such that two offset radiopaque markers 2401A, 2401B are visible on the first end ring 110A. The indirect or S-shaped connectors 2802 between adjacent windings are shown in FIGs. 32A-B. Links 119 connecting the interconnected bands 115A-B of an individual winding are also shown in FIGs. 32A-B. FIG. 33 illustrates a perspective or 3D view of the stent 100 shown in FIGs. 32A-B. FIG. 34 is identical to the stent 100 shown in FIG. 32B, except FIG. 34 is shown without the inner tube, which may represent an inner mandrel used during stent manufacture, or an inner guide catheter tubing or inner guidewire used during stent delivery, for example.

It should be noted, that in any of the embodiments of the present invention, the stent 100 may include the bent strut design in all, some or one strut depending, for example, on the particular application and/or desired expanded or compressed stent diameter size. Alternatively, in any of the embodiment of the present invention, the stent 100 may not include the bent strut design, but rather may include a linear or straight strut design in all the struts of the undulating pattern. Such a linear strut design may be used in combination with any one or more of the other features described above, such as in combination with at least one of the features of the main stent component, end rings, polymer coating, controlled drug elution, staggered pattern of alignment of adjacent loops, offset radiopaque markers, unconnected adjacent windings in the longitudinal direction, H-shaped connected adjacent windings, and/or S-shaped connected adjacent windings, for example. Similarly, any of the features of the stent 100 described in any one of the embodiments above may be incorporated in any stent individually or in combination with any one or more of the features described above. For example, the polymer coating, controlled drug elution, bent strut design, staggered pattern of alignment of adjacent loops, offset radiopaque markers, unconnected adjacent windings, H-shaped connected adjacent windings, and/or S-shaped connected adjacent windings described above may individually or in any combination be incorporated into any stent, and is not limited to the stent 100 described above. That is, while the above described features of stent 100 are illustrated on a helical, spiral or coiled stent design, it is expressly noted that such above described features may alone or in combination be incorporated into any other type of stent design, such as a ring stent design which does not include a continuously wound pattern but rather includes a series of separate, unconnected and closed rings.

It is also noted that while the structural components of the stent of the present invention are not separate structures and are formed integral to each other (via, *e.g*., laser cutting or chemical etching) to form the continuous tubular shape of the helical (e.g., spiral or coiled) stent of the present invention or to form the noncontinuous tubular shape of the ring stent of the present invention, the structural components, such as the interconnected bands, struts, loops, links, and/or end rings, among other features have been referred to separately for ease of identification and discussion. Further, it should be noted that reference to the same reference numerals in different drawings indicate the same features.

It should be understood that the above description and drawings are only representative of illustrative examples of embodiments. For example, it is understood that the bent strut design described herein, which contributes to the nestled arrangement for reducing the compressed diameter of the stent, may be incorporated in any appropriate intraluminal endovascular devices (*e.g.*, a stent, graft or stent-graft device) as desired, including, for example, a stent having any number of bands within a winding. For the reader's convenience, the above description has focused on a representative sample of possible embodiments, a sample that teaches the principles of the invention. Other embodiments may result from a different combination of portions of different embodiments. The description has not attempted to exhaustively enumerate all possible variations.

## Claims

1. An endovascular device (100), comprising:
a main stent component (105) having a tubular shape, a first end (105a) and a second end (105b), wherein the main stent component (105) comprises:
a helical stent pattern having a plurality of continuous windings such that the plurality of windings (115) are oriented in a helical direction (H) of the endovascular device (100),
wherein each winding of the plurality of windings (115) comprises two interconnected bands (115A-B), and
wherein at least two adjacent windings (115) are interconnected in the longitudinal direction of the stent by a flexible connection (2702, 2802).

2. The endovascular device (100) of claim 1, wherein the windings (115) have an undulating pattern comprising struts (120) and loops (125), the loops (125) being portions of the undulating pattern having a turn of about 180 degrees, wherein each end of a loop (125) is coupled to an end (120c) of a strut (120) forming a pair of struts (122), and wherein adjacent loops (125) within each winding (115) are axially offset with respect to a perpendicular axis perpendicular to the lengthwise direction (L) to form a staggered pattern of alignment (A) of adjacent loops such that a loop (125c) is positioned to align with an end of an adjacent strut (120c) within each winding (115).

3. The endovascular device (100) of claim 2, wherein the flexible connection is an indirect connector (2802) connecting adjacent struts (120) of adjacent windings, wherein the indirect connector (2802) has a curved pattern.

4. The endovascular device (100) of claim 2 or 3, wherein the flexible connection is a direct connector (2702) directly connecting adjacent loops (125) of adjacent windings.

5. The endovascular device (100) of any one of claims 2 to 4, wherein at least one strut (120) is a bent strut comprising a bent pattern (130) along a length of the bent strut (120) in the crimped delivery diameter.

6. The endovascular device (100) of any one of claims 1 to 5, further comprising a first radiopaque marker (2401A) having a shape with at least two distinct profiles when viewed from different angles, said first marker (2401A) being attached to the main stent component (105), and
a second radiopaque marker (2401B) having a shape with at least two distinct profiles when viewed from different angles, said second marker (2401B) being attached to the main stent component (105), wherein the first and second radiopaque markers (2401A, 2401B) are positioned on the main stent component to be offset by less than 180 degrees relative to the other.

7. The endovascular device (100) of any one of claims 1 to 6, wherein the main stent component (105) is made of cobalt chromium (CoCr), and further comprises a polymer mesh (200) covering portions of the main stent component (105), the polymer mesh (200) comprising a biodegradable material selected from the group consisting of DL-lactide/glycolide copolymer (PDLG), poly-DL-lactide (PLC), and combination thereof, and wherein the polymer mesh (200) further comprises ridaforolimus.
